(19) Europäisches Patentamt / European Patent Office / Office européen des brevets

(11) **EP 3 111 833 B1**

(12) **EUROPEAN PATENT SPECIFICATION**

(45) Date of publication and mention of the grant of the patent:
**27.11.2024 Bulletin 2024/48**

(21) Application number: **16181509.7**

(22) Date of filing: **30.01.2015**

(51) International Patent Classification (IPC):
**A61B 5/00** (2006.01) **A61B 5/1459** (2006.01)

(52) Cooperative Patent Classification (CPC):
**A61B 5/0066; A61B 5/7207; G01B 9/02091; G01B 9/02094; G01P 5/26; G01S 7/4808; G01S 17/58; G01S 17/66; G01S 17/88; G01S 17/89;** A61B 5/1459; A61B 2562/0233; A61B 2576/00; G01P 21/02; G16H 30/40

(54) **METHOD AND APPARATUS FOR PERFORMING MULTIDIMENSIONAL VELOCITY MEASUREMENTS BASED ON AMPLITUDE AND PHASE SIGNALS IN THE FIELD OF OPTICAL INTERFEROMETRY**

VERFAHREN UND VORRICHTUNG ZUR DURCHFÜHRUNG VON MEHRDIMENSIONALEN GESCHWINDIGKEITSMESSUNGEN MIT OPTISCHER INTERFEROMETRIE

PROCÉDÉ ET APPAREIL PERMETTANT D'EFFECTUER DES MESURES DE VITESSE MULTIDIMENSIONNELLES À L'AIDE DES SIGNAUX D'AMPLITUDE ET DE PHASE DANS LE CADRE DE L'INTERFÉROMÉTRIE OPTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.01.2014 US 201461933965 P**

(43) Date of publication of application:
**04.01.2017 Bulletin 2017/01**

(62) Document number(s) of the earlier application(s) in accordance with Art. 76 EPC:
**15000292.1 / 2 932 889**

(73) Proprietor: **The General Hospital Corporation Boston, MA 02114 (US)**

(72) Inventors:
- **Uribe-Patarroyo, Nestor Brookline, MA 02446 (US)**
- **Vakoc, Benjamin Arlington, MA 02474 (US)**
- **Eugene Bouma, Brett Quincy, MA 02171 (US)**
- **Villiger, Martin Cambridge, MA 02139 (US)**

(74) Representative: **Maiwald GmbH Elisenhof Elisenstraße 3 80335 München (DE)**

(56) References cited:
**WO-A2-2009/137701**

- **MURTAZA &NDASH; ALI ET AL: "White Paper Signal Processing Overview of Optical Coherence Tomography Systems for Medical Imaging", SIGNAL PROCESSING OVERVIEW OF OPTICAL COHERENCE TOMOGRAPHY SYSTEMS FOR MEDICAL IMAGING, 30 June 2010 (2010-06-30), XP055192967, Retrieved from the Internet <URL:http://www.ti.com.cn/cn/lit/wp/sprabb9/sprabb9.pdf> [retrieved on 20150602]**
- **REZA MOTAGHIANNEZAM ET AL: "Logarithmic intensity and speckle-based motion contrast methods for human retinal vasculature visualization using swept source optical coherence tomography", BIOMEDICAL OPTICS EXPRESS, vol. 3, no. 3, 1 March 2012 (2012-03-01), pages 503 - 682, XP055209667, ISSN: 2156-7085, DOI: 10.1364/BOE.3.000503**

- MACIEJ WOJTKOWSKI: "High-speed optical coherence tomography: basics and applications", APPLIED OPTICS, OPTICAL SOCIETY OF AMERICA, WASHINGTON, DC; US, vol. 49, no. 16, 1 June 2010 (2010-06-01), pages D30 - D61, XP001554563, ISSN: 0003-6935, DOI: 10.1364/AO.49.000D30
- JONGHWAN LEE ET AL: "Dynamic light scattering optical coherence tomography", OPTICS EXPRESS, vol. 20, no. 20, 13 September 2012 (2012-09-13), pages 22262 - 585, XP055321344, DOI: 10.1364/OE.20.022262
- NICOLÁS WEISS ET AL: "Localized measurement of longitudinal and transverse flow velocities in colloidal suspensions using optical coherence tomography", PHYSICAL REVIEW E (STATISTICAL, NONLINEAR, AND SOFT MATTER PHYSICS), vol. 88, no. 4, 1 October 2013 (2013-10-01), US, XP055321291, ISSN: 1539-3755, DOI: 10.1103/PhysRevE.88.042312
- VIVEK J. SRINIVASAN ET AL: "Quantitative cerebral blood flow with Optical Coherence Tomography", OPTICS EXPRESS, vol. 18, no. 3, 1 February 2010 (2010-02-01), pages 2477, XP055229966, DOI: 10.1364/OE.18.002477

## Description

The present application relates to and claims priority from U.S. Provisional Patent Application Serial No. 61/933,965 filed January 30, 2014.

## FIELD OF THE DISCLOSURE

Exemplary embodiments of the present disclosure relate generally to optical measurements of velocities, and more particularly to methods, systems and apparatus for providing multidimensional velocity measurements using amplitude and phase information in an optical interferometry.

## BACKGROUND INFORMATION

Optical interferometric techniques to measure velocities can rely on the Doppler effect. The Doppler effect can describe a change in the frequency of light when it is reflected from a moving object. This permits the determination of the relative velocity of a sample, multiple samples or different parts of a sample with respect to the illuminating probe when it is shined with radiation that is analyzed in the frequency domain after reflection.

In some fields, it can be important to know a velocity map of a sample, for example when viscoelastic objects move with time. In these cases, the object can be considered as being composed of different parts that move independently, and therefore it can be valuable to make use of the Doppler effect in optical techniques that have higher imaging dimensionality, such as 2D and/or 3D imaging with a depth discrimination, so as to obtain a complete profile of the velocity distribution of the object of interest. In particular, Doppler Optical Coherence Tomography (D-OCT) is a technique known in the art for determining the velocity distribution as a function of depth in biological or other types of samples, with the potential of providing 2D and 3D velocity profiles when combined with multidimensional imaging or scanning systems. An optical system capable of performing D-OCT measurements consists of a phase-stable interferometer and data acquisition system that is able to detect changes in phase with time that come exclusively from the sample, without phase errors induced by fluctuations in the interferometer itself or by the acquisition process. D-OCT is very sensitive but has a limited range, being able to detect velocities as low as tens of micrometers per second up to millimeters per second. In the limited cases in which the sample of interest has a smooth velocity pattern and the spatial resolution of the Doppler phase map is sufficiently high, phase unwrapping algorithms can be used to extend the limits of the velocity range.

Currently, the fastest OCT imaging systems can be provided with a light source that sweeps the optical Fourier domain, known as Optical-Frequency Domain Imaging (OFDI) systems. The sources used on OFDI impose stringent requirements on the timing of the data acquisition, which may not be easy to satisfy in order to have a phase stable measurement, due to drift in the timing clocks of the high-frequency data acquisition systems used in OFDI. Some solutions for this problem have been proposed, but they usually add important complexity to the light source, such as an additional interferometer with its own data acquisition system, This has limited the mainstream implementation of D-OCT in swept-source systems, which has hindered the use of OFDI for the fast measurement of multidimensional velocity profiles in many fields.

D-OCT procedures and/or configuration are capable of establishing the sign of the direction of the movements, but can be directly sensitive only to movement in the line of sight (LOS). This has been another limitation for the use of D-OCT systems, because the angle that the D-OCT light beam makes with the moving object must be known in order to accurately determine the velocity. When the sample of interest has a map of velocities with varying orientation, such as those found in the most general flow of a liquid, it is not possible to know the angle of the velocities with respect to the light beam without *a priori* knowledge of the complete vectorial distribution of the velocities that the measurement intends to determine. Errors in the knowledge of this angle translate into errors in the measured velocities. This has been a very important drawback of the D-OCT method that makes it difficult to use in applications where an accurate description of the velocity profile is needed.

For example, one of these applications consists of determining the velocity profile in the blood flow inside blood vessels, and determining the total flow rate. Only when the flow is well-behaved unidirectional laminar flow, D-OCT is able to quantify the flow rate. Unfortunately, these conditions are rarely met in biological tissue. This inhibits, in principle, the use of D-OCT for characterizing blood flow inside blood vessels with branches and ramifications, as well as its use for quantifying the total flow rate in these conditions. For the reasons described above, it would be highly desirable to have a method that is able to determine velocity distributions without a prior knowledge of the velocity directional distribution.

There have been proposals for the use of Doppler analysis to determining a movement out of the LOS direction, in particular the use of the Doppler variance. However, Doppler variance is strongly linked with the signal-to-noise ratio (SNR) and the focusing optics, and depends on zero or a calibrated constant variance in the LOS direction, which makes it undesirable for use in real-world scenarios.

Another set of techniques use different quantities based on the speckle that forms when radiation is scattered from the sample in a coherent imaging system. In a coherent optical or acoustic system, speckle forms due to the interference between multiple signals scattered from different parts of the object under study inside the resolution volume, and speckle evolves as a stochastic process whose statistics are related to the movement of the scatterers. Speckle-based techniques do not rely on the phase information of the signal, only on the statistical fluctuations of speckle intensity. For example, it is possible to relate the variance of the speckle above a given threshold to the presence of moving scatterers in the sample, such as those occurring in a flowing liquid. However this technique is purely qualitative, as it is only capable of discriminating moving and static areas. Other speckle techniques are based on cross-correlation between speckle taken at different times, or on the time autocorrelation of speckle. Although in this case it is theoretically possible to quantify speed, so far there have been no known reliable systems for the measurement of speed distributions based on speckle statistics.

Dynamic light scattering (DLS) is a technique that analyzes statistics of complex OCT speckle. DLS can obtain quantitative information regarding the LOS and transverse motion of the scatterers by analyzing the complex speckle signal, but it relies on the phase of the OCT signal. Therefore, DLS requires a phase-stable OCT system to determine vectorial speed profiles. The use of the complex signal is at the core of the technique, which cannot be separated into distinct amplitude and phase analyses. It can be highly valuable to provide technique, system, method, apparatus and/or computer-accessible medium that can carry out the same measurements of DLS, depending only on the amplitude of the OCT signal.

Gradients in the LOS velocity can likely impact speckle statistics, and the effects can be severe when the velocity is mainly aligned. A known technique for addressing this problem relies on measuring the axial velocity using the phase information of the signal. However, this makes it difficult to make quantitative measurements when only the amplitude of the signal is available. It can be valuable to provide technique, system, method, apparatus and/or computer-accessible medium that, having access to only the amplitude signal, can determine the axial gradient distribution and to compensate for its effects on the decorrelation of the signal. Bringing the speckle correlation techniques to obtaining quantitative measurements would be useful for the widespread use of coherent systems for determining speed distributions, and could enable the use of OFDI systems for multidimensional velocity measurements.

Speckle techniques can be sensitive to speed in any direction, but no technique, system, method, apparatus and/or computer-accessible medium based on amplitude speckle statistics have been able to determine the axis in which the movement occurs. It would be valuable to develop a speckle technique that, although sensitive to velocity in any direction, is at the same time able to discriminate between flow in the LOS and out of the LOS. For example, in the viscous flow of a liquid, turbulence can appear at some regimes and the determination of the level of turbulence is highly valuable in some fields of study. Doppler would not be able to identify the areas with turbulent flow as it only identifies flow in the line of sight. Current speckle techniques could potentially be sensitive to the turbulent flow but cannot provide the directional information that is necessary to assess the total flow rate, which produces an overestimation of the total flow rate.

Apart from tracking the motion of a sample, in some areas, it can be of interest to track the motion of the probe that is being used to measure the sample interferometrically. In some fields, the motion of the probe cannot be completely controlled, with unexpected discontinuities during the scanning process. These discontinuities can appear in translating and rotating degrees of freedom, and without correction, can severely distort the images acquired. It would be valuable to provide technique, system, method, apparatus and/or computer-accessible medium that can use speckle amplitude statistics and/or phase to track the motion of the probe so as to compensate for image distortion.

Accordingly, there may be a need to address and/or overcome at least some of the issues of deficiencies described herein above.

Indeed, based on the above, it would be desirable to provide system, apparatus, methods and techniques that overcomes the limitations of the phase-based velocity measurements in high-speed interferometry systems, and address the line-of-sight problems of Doppler techniques, and overcome the lack of reliable quantitative and directionality information in intensity-based speckle speed measurements.

The scientific articles "White Paper Signal Processing Overview of Optical Coherence Tomography Systems for Medical Imaging" (Murtaza, ET AL, 2010-06-30, XP055192967) and "Logarithmic intensity and speckle-based motion contrast methods for human retinal vasculature visualization using swept source optical coherence tomography", (Motaghiannezam ET AL, 2012-03-01, XP055209667, DOI: 10.1364/ BOE.3.000503) are pertinent to the understanding of the present invention. Furthermore, the following patent application is also pertinent to the understanding of the present invention: WO 2009/137701 A2 (COLICE ET ALL , 2009-11-12).

SUMMARY

A first embodiment of the invention is the optical frequency domain imaging apparatus defined in claim 1. A second embodiment of the invention is the optical frequency domain imaging method defined in claim 3. Preferred embodiments of the apparatus are defined in claims 2, 4 and 5. Preferred embodiments of the method are defined in claims 6 and 7.

## BRIEF DESCRIPTION OF THE DRAWINGS

Further objects, features and advantages of the present disclosure will become apparent from the following detailed description taken in conjunction with the accompanying figures in which:

Figure 1 is a block diagram of a conventional coherent imaging system;

Figure 2 is a block diagram of a conventional interferometric imaging system;

Figure 3 is a block diagram of a conventional optical frequency-domain imaging system using a coherent single-frequency tuning source;

Figure 4 is a block diagram of a conventional optical frequency-domain imaging system using a coherent single-frequency tuning source in accordance with an exemplary embodiment of the present disclosure;

Figure 5 is an conventional optical coupling and scanning mechanism in the form of a medical catheter for measuring flow profiles of a cylindrical sample such as a flow velocity inside a blood vessel;

Figure 6 is a flow diagram of a method for correcting the phase using a reference sample phase signal in accordance with in accordance with an exemplary embodiment of the present disclosure;

Figure 7 is a flow diagram of a method for determining the speed profile of a sample using amplitude speckle statistics in accordance with an exemplary embodiment of the present disclosure;

Figure 8 is a set of illustrations of a conventional OFDI intensity measurement of a flowing liquid;

Figures 9A-9C are exemplary illustrations of inverse correlation time profiles for a moving solid as measured using the conventional autocorrelation method;

Figures 9D-9F are exemplary illustrations of the exemplary inverse correlation time profiles for the moving solid as measured by the exemplary embodiment of the speckle-amplitude statistics OFDI system according to an exemplary embodiment of the present disclosure;

Figure 10 is an illustration of an exemplary parametrization of speed as a function of $\tau^{-1}$ and SNR based on the present exemplary material as measured by the exemplary embodiment of the speckle-amplitude statistics OFDI system according to an exemplary embodiment of the present disclosure;

Figure 11 is a set of exemplary illustrations of speed profiles for a moving solid phantom as measured using the exemplary parametrization of speed as a function of $\tau^{-1}$ and SNR based on the present exemplary material as measured by the exemplary embodiment of the speckle-amplitude statistics OFDI system according to an exemplary embodiment of the present disclosure;

Figures 12A-12C are exemplary illustrations of speed profiles for a flowing liquid as measured by the exemplary embodiment of the speckle-amplitude statistics OFDI system according to an exemplary embodiment of the present disclosure;

Figures 12D-12F are exemplary illustrations of the flow speed as measured by Doppler analysis using the exemplary embodiment of the phase-correction OFDI system according to an exemplary embodiment of the present disclosure;

Figures 13A-13C are exemplary illustrations of the conventional structural image from OFDI of the tube and the liquid;

Figures 13D-13F are exemplary illustrations of two-dimensional speed profiles for a flowing liquid as measured by the exemplary embodiment of the speckle-amplitude statistics OFDI system and method according to an exemplary embodiment of the present disclosure;

Figures 13G-13I are exemplary illustrations of two-dimensional flow speeds as measured by Doppler analysis using the exemplary embodiment of the phase-correction OFDI system and method according to an exemplary embodiment of the present disclosure;

Figures 14A-14C are exemplary illustrations of three-dimensional speed profiles for a flowing liquid at different planes in the tube longitudinal direction as measured by the exemplary embodiment of the speckle-amplitude statistics OFDI system and method according to an exemplary embodiment of the present disclosure;

Figures 14D-14F are exemplary illustrations of three-dimensional speed profiles for a flow velocity measured by Doppler analysis using the exemplary embodiment of the phase-correction OFDI system and method according to an exemplary embodiment of the present disclosure;

Figures 14G-14I are exemplary illustrations of conventional structural images from OFDI of the tube and the liquid;

Figure 15 is a flow diagram of a method for determining a vectorial speed and a turbulence profile of the sample using at least two amplitude speckle statistics analysis with different scanning speeds in accordance with an exemplary embodiment of the present disclosure;

Figure 16A is a flow diagram of a method for determining figures or areas of interest, such as axial and/or lateral speed profile, and/or an axial speed gradient of the sample using multiple amplitude speckle statistics analyses, e.g., with different k-space signal diversities in accordance with an exemplary embodiment of the present disclosure;

Figure 16B is a set of exemplary illustrations of axial velocity gradient determination using the exemplary embodiment of the amplitude speckle statistics analysis using k-space signal diversity system and method according to the present invention;

Figure 16C is a set of exemplary illustrations of axial and lateral speed determination using the exemplary embodiment of the amplitude speckle statistics analysis using k-space signal diversity system and method according to the present invention;

Figure 17 is a flow diagram of a method for determining the vectorial speed profile of the sample using a combination of Doppler and amplitude speckle statistics analysis in accordance with an exemplary embodiment of the present disclosure;

Figure 18 is a flow diagram of a method for determining the turbulence profile of the sample using a combination of Doppler and amplitude speckle statistics analysis in accordance with an exemplary embodiment of the present disclosure;

Figure 19 is a flow diagram of a method for tracking the probe translational motion using Doppler analysis in accordance with an exemplary embodiment of the present disclosure;

Figure 20 is a flow diagram of a method for tracking the probe rotational motion using amplitude speckle statistics analysis in accordance with an exemplary embodiment of the present disclosure;

Figure 21 is a set of exemplary illustrations of translational probe motion tracking using the exemplary embodiment of the Doppler probe tracking system and method according to the present disclosure; and

Figure 22 is a set of exemplary illustrations of rotational probe motion tracking and image correction using the exemplary embodiment of the amplitude speckle statistics analysis rotational probe tracking system and method according to the present disclosure.

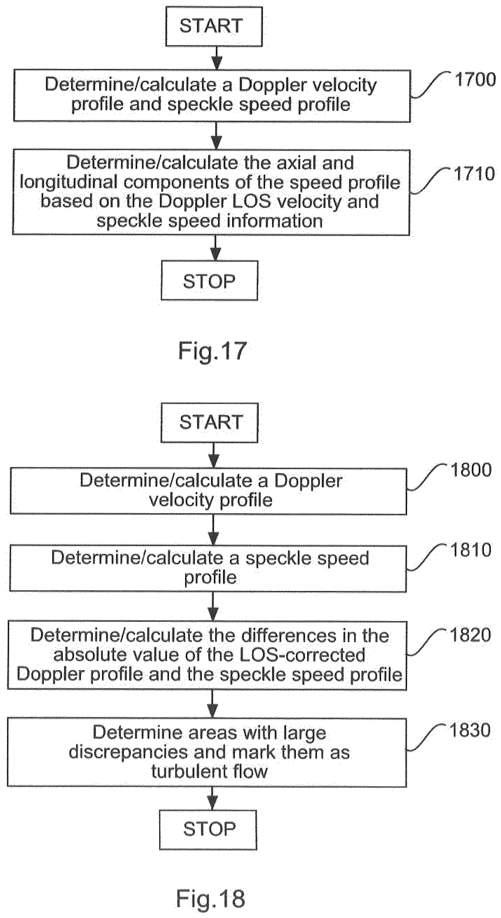

Figure 18 illustrates the invention defined in the appended claims.

Throughout the figures, the same reference numerals and characters, unless otherwise stated, are used to denote like features, elements, components or portions of the illustrated embodiments. Moreover, while the subject disclosure will now be described in detail with reference to the figures, it is done so in connection with the illustrative embodiments.

**DETAILED DESCRIPTION OF EXEMPLARY EMBODIMENTS**

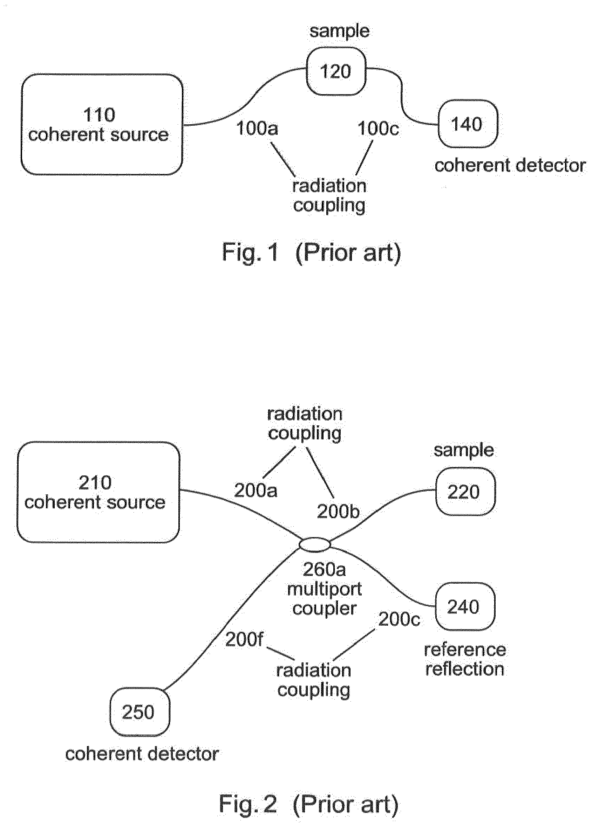

Referring to Figure 1, a conventional coherence imaging system can include a coherent source 110 which provides a temporally coherent electromagnetic or acoustic signal to a sample 120 and a reference sample 130. The radiation propagates through the radiation coupling 100a, which can consist of free-space components or of wave-guiding components. 100a can be fitted with means to perform multidimensional imaging, such as lenses or scanning systems. After radiation is scattered by 120 one more radiation coupling component 100c transmits the radiation to a coherent detector 140. Due to the coherent nature of the source and of the detection, when the sample 120 includes many subresolution scatterers, the image of sample 120 can likely be composed of speckle.

It is possible to analyze the statistical variations of the speckle amplitude in order to gain information on the movement of the sample. Such techniques usually rely on implementing 100a as a two-dimensional detector, and the analysis performed on the acquired amplitude of speckle as a function of time is known as laser speckle contrast imaging. These techniques usually provide qualitative information on whether a part of the object is moving or not, but determining actual speeds is not possible.

Referring now to Figure 2, an exemplary interferometric imaging system can include a coherent source 210 which can provide an electromagnetic and/or acoustic signal to a multiport coupler 260a. This coupler 260a, in the case of the electromagnetic radiation, can be a beam splitter, as is generally known in the art. The radiation can propagate through a radiation coupling 200a, which can include free-space components or of wave-guiding components. After the radiation coupling 260a, the radiation can be separated in two couplings 200b and 200c. Radiation going into the coupling 200b is delivered to the sample 220. The coupling 200d can be fitted so as to perform multidimensional imaging, such as lenses or scanning systems. After radiation is scattered by 220, radiation is coupled back into the coupling 200b and transmitted through a multiport coupler 260a. Radiation is also delivered through 200c to the reference reflection 240, which can have means to change the effective optical path length as is known in the art. Coherent radiation reflected by the sample 220 and the reference 240 can be mixed by multiport coupler 260a and delivered by a coupler 200f into a coherent detector 250. Due to the coherent mixing of the signals, by appropriate measurement schemes as it is known in the art, it is possible to determine both the amplitude and phase of the radiation reflected from sample 220. In the case that sample 220 can include many subresolution scatterers, the amplitude and phase detected can present speckle.

It is possible to perform a Doppler analysis on the phase variations of the reflected radiation in order to gain information on the movement of the sample. Such techniques are generally only sensitive to the LOS movement of the sample, which inhibits the reconstruction of a complete velocity profile. As Doppler can be sensitive to the sign of the movement, movement that occurs in many directions in a determined region of the sample (such as that movement that appear in the turbulent flow of a liquid) may not be accurately determined, and such regions with rapid velocity direction changes cannot be identified. Furthermore, Doppler techniques have a defined limit given by the phase wrapping effect.

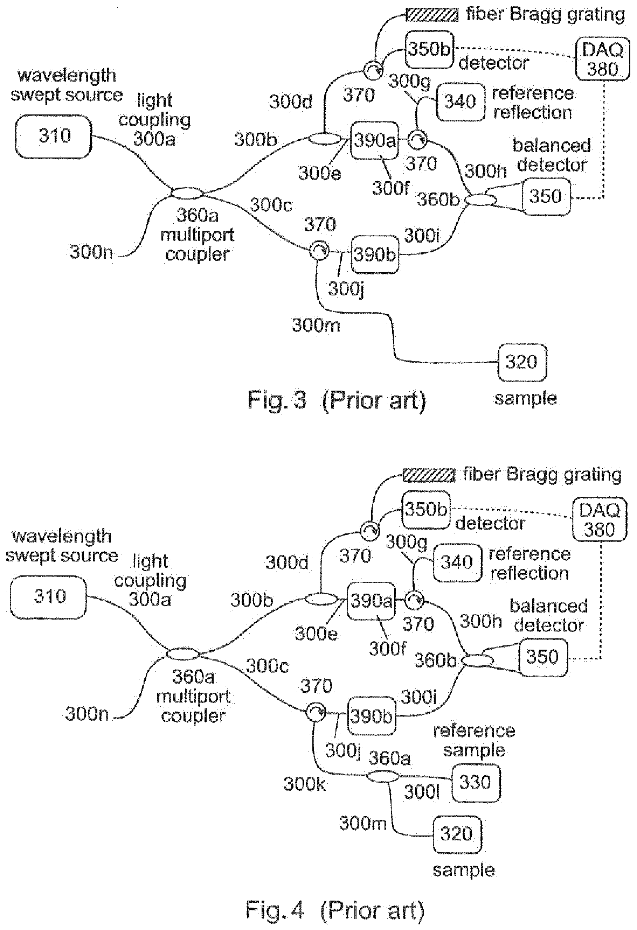

Referring now to Figure 3, an exemplary OFDI imaging system is shown as a block diagram that can include a wavelength-swept coherent source 310 which can provide an electromagnetic signal to a multiport coupler 360a. This coupler 360a can be or include a beam splitter, as is generally known in the art. The radiation propagates through the radiation coupling 300a, which can consist of free-space components or of wave-guiding components. After the multiport coupler 360a, the radiation can be separated in two couplings 300b and 300c, which can comprise a sample and a reference arm, respectively. The radiation provided into a coupling 300b can be further split into couplers 300d and 300c. The coupler 300d can provide the radiation to a fiber Bragg grating that, upon going to an optical circulator 370, provides a clock signal at a detector 350b that can be further digitized in a data acquisition (DAQ) system 380. The portion of light/radiation that continues through a path 300e feeds the polarization and/or frequency shifter 390a for polarization diverse sensing and/or for removal of the depth degeneracy, as is well known in the art. Coupling 300f can feed a circulator 370 to provide radiation to the reference reflection 340 via a coupler 300g, and the coupler/path 300h delivers reflected light/radiation into a multiport coupler 360b.

Similarly, the sample arm is comprised of circulator 370 that collects light reflected from sample 320, which is delivered via 300k. Coupling 300j can guide the radiation into the polarization and/or frequency shifter 390b which are used as it is well known in the art. Coupling 300i can deliver the sample light/radiation into the multiport coupler 360b, which can mix light/radiation from the reference reflection, reference sample and sample, and balance detector assembly 350 consists of detectors and optionally multiport couplers to have polarization sensitive detection. 300k can be fitted with means to perform multidimensional imaging, such as lenses or scanning systems. The reference reflection 340 can have a way to change the effective optical path length as is known in the art. A signal from 350 can be digitized by the data acquisition board (DAQ) 380. Due to the coherent mixing of the signals, by appropriate measurement schemes, it is possible to determine both the amplitude and phase of the radiation reflected from the sample 320 as a function of depth. In the case that sample 320 consists of many subresolution scatterers, the amplitude and phase detected will present speckle.

It is possible to perform a Doppler analysis on the phase variations of the reflected radiation in order to gain information on the movement of the sample in a similar way to the exemplary embodiment shown in Figure 2. The same or similar limitations can apply, e.g., the sensitivity to only LOS movement of the sample, the inability to accurately determine regions with movement in many directions (such as that movement that appear in the turbulent flow of a liquid) and the well-defined limit given by the phase wrapping effect.

Figure 4 shows an exemplary OFDI imaging system according to an exemplary embodiment of the present disclosure with a similar configuration to the conventional system depicted in Figure 3, configured to further subdivide the radiation in the light coupling 300k into couplings/paths 300l and 300m due to a multiport coupler 360a.

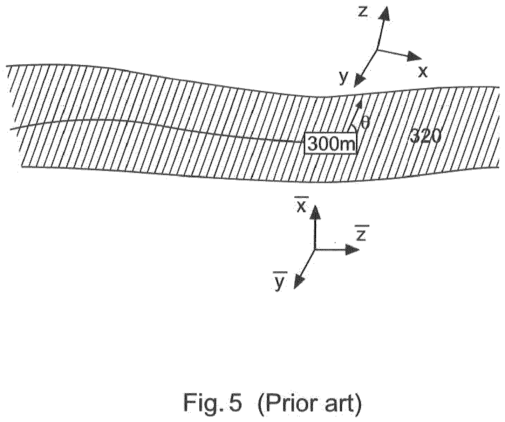

For example, as shown in Figure 4, the light coupling and scanning system 300m can have many implementations. For example, in the case of OFDI systems used in cardiovascular applications, the exemplary system 300m can be configured and/or provided in the form of a catheter that is well known in the art, as shown in Figure 5. The sample 320, e.g., in cardiovascular applications, can be or include blood inside a vessel, the vessel wall itself, or a combination of both. An exemplary scanning system based on a catheter can perform one-dimensional measurements of the reflectance of the sample with depth (known as A-lines), and mechanical rotation allows for scanning of the transversal plane. At the same time the catheter can be "pulled-back" to provide sectioning along the longitudinal direction to gather three-dimensional data from the sample.

## EXEMPLARY CORRECTION OF THE ABSOLUTE PHASE DUE TO PHASE INSTABILITIES IN INTERFEROMETER OR DATA ACQUISITION SYSTEM

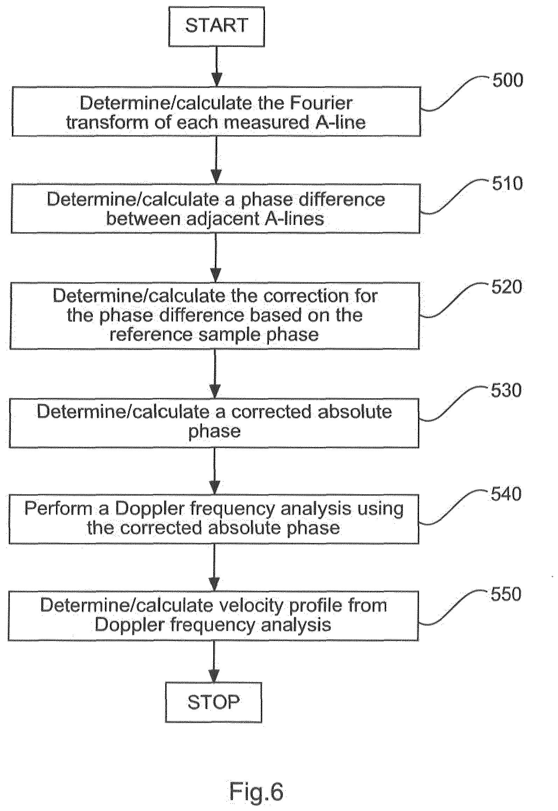

Figure 6 shows a flow diagram of an exemplary embodiment of a method for correcting the absolute phase acquired from the sample using the exemplary embodiment of Figure 4. An exemplary embodiment for this correction is as follows:

1) Determine/calculate the axial complex-valued profile of each measured A-line $a_{km} = \mathrm{DFT}(S_{km})$, where $k$ denotes the depth index, $m$ the time index and DFT a discrete Fourier transform function. The time index can optionally be coupled with a spatial scanning (step 500).

2) Determine/calculate an absolute phase $\varphi_{km} = \arg(a_{km})$, where arg denotes the argument of the complex number $a_{km}$ (step 510).

3) Determine/calculate the correction for the phase difference $\Delta_{km} = \phi_{k,m+1} - \phi_{km}$ based on the reference sample(s) phase (330 in Figure 4). Defining the phase correction $\delta_{km}$, the corrected phase difference is $\tilde{\Delta}_{km} \equiv \Delta_{km} + \delta_{km} = \phi_{k,m+1} - \phi_{km} + \delta_{km}$. There are different types of phase corrections. There are cases in which an offset correction is necessary. For example when element 390a and/or 390b contains a frequency shifter, or when the scanning probe 300k is subject to unwanted vibrations. To correct an offset, the reference sample phase change $\Delta_{k',m}$ at depth $k'$ is known and the corrected phase difference is $\tilde{\Delta}_{km} = \Delta_{k,m} - \Delta_{k',m}$ so $\delta_{km} = -\Delta_{k',m}$. When there is timing-induced phase jitter the correction is well known in the art: for instance, when a reference reflection at depth $k'$ is used, the correction has the form $\delta_{km} = -k/k'\Delta_{k',m}$. When several phase artifacts are present they can be corrected accumulatively, and might need more than one reference reflection. For example, first correcting an offset correction due to a frequency shifter, secondly correcting a timing-induced jitter slope, and thirdly correcting an offset due to vibration of the scanning probe 300m (step 520).

4) Determine/calculate a corrected absolute phase based on the correction for the phase differences by

$$\tilde{\varphi}_{km} = \varphi_{km} + \sum_{n=1}^{m} \delta_{k,n+1}$$

. A phase offset as a function of depth for the first A-line $m = 1$ will be present that cannot be compensated, which does not alter the results of the next step (step 530).

5) Perform a Doppler frequency analysis using the corrected absolute phase. As it is well known in the art, this analysis can use Fourier analysis or autocorrelations such as Kasai autocorrelation. These methods only work on the absolute phase, not on the phase differences. These methods provide a robust way to calculate the Doppler mean frequency as well as other figures of interest such as Doppler frequency variance (step 540).

6) Determine/calculate velocity profile from Doppler frequency analysis, where the Fourier analysis is performed at different regions of the sample image in order to extract a multidimensional velocity profile (step 550).

In the exemplary embodiment of the exemplary systems shown in Figures 4 and 5, it is possible to define two coordinate systems to which the velocity profiles can be referred. It should be understood that other exemplary embodiments can be used to determine multidimensional velocity profiles in accordance with an exemplary embodiment of the present disclosure. For example, the first coordinate system can define $z$ as the light propagation direction, and its associated perpendicular $xy$ plane. The second system can be defined with respect to the sample 320, which in case of the exemplary embodiment shown Figure 5 has a cylindrical shape, however it is not limited in any way to this geometry. In this exemplary coordinate system, $\tilde{z}$ is looking forward in the longitudinal direction of the catheter and the $\tilde{xy}$ defines the plane of rotation of the beam. It is possible to define $x$ and $\tilde{z}$ as parallel, so an ideal laminar flow inside the tube flows along the *x* direction which is independent of the rotation angle $\theta$ of the beam, while the *y* direction depends on $\theta$.

## NOISE-TOLERANT SPECKLE-AMPLITUDE AUTOCORRELATION TECHNIQUE

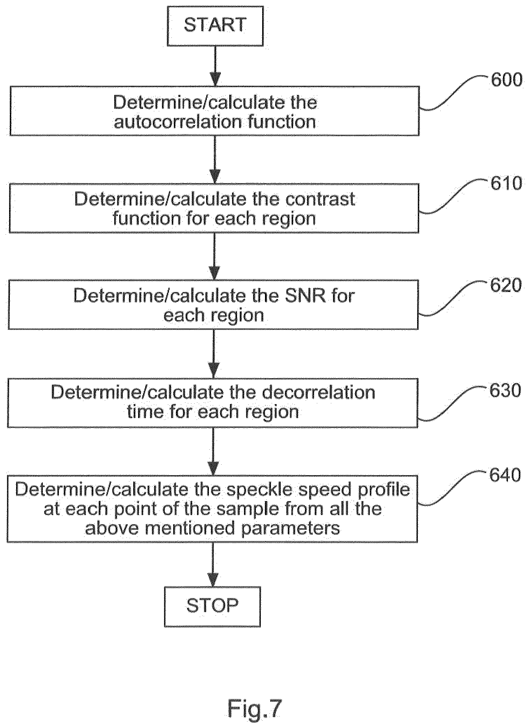

Figure 7 shows a flow diagram of an exemplary embodiment of a method according to the present disclosure for calculating and/or otherwise determining a speed profile based on an analysis of the autocorrelation of the speckle amplitude acquired from the sample. The steps/procedures are explained in detail herein below.

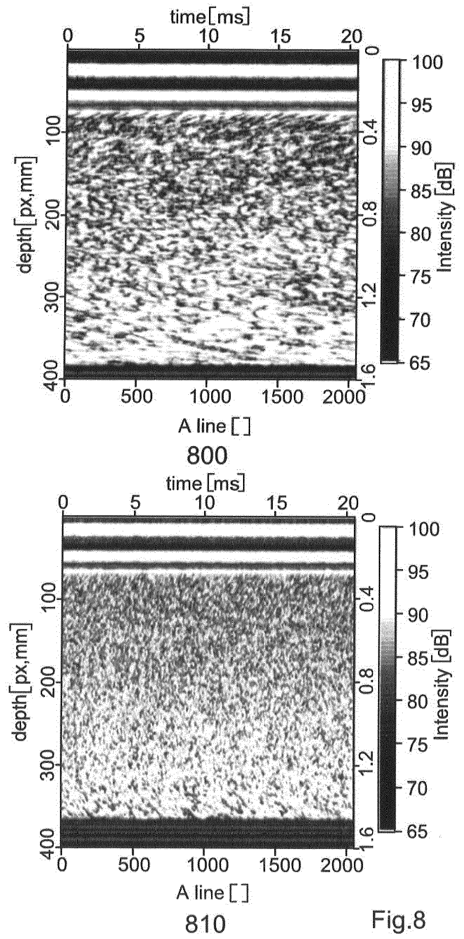

In a coherent optical system where fully developed speckle is formed after reflection of light by moving particles, the speckle-decorrelation time is inversely proportional to the speed of the particles. In the case of OFDI, there is a speckle size in the axial direction that can be related to the axial resolution of the system (given by the bandwidth of the light source). At each depth, the speckle can evolve as a function of time so that its decorrelation time is inversely proportional to the particles reflecting light at that depth. Figure 8 shows a set of illustrations providing examples of this relation, where the tomogram of a tube is shown that is filled with flowing scattering liquid (e.g., intralipid) at two different flow rates. Reference 800 in Figure 8 indicates an M-mode tomogram of phantom fluid at 2.5 mL / min with low flow velocities and large speckle size, while reference 810 in Figure 8 shows the same sample at 80 mL / min yielding high flow speeds and small speckle size. These tomograms are so called M-mode measurements, which consist of the measurement of A-lines as a function of time of the same position in the sample.

Thus, the horizontal axis shown in Figure 8 corresponds to time, while the vertical axis to depth. The catheter is provided at the top, clearly identified by the strong reflections of the collimating ball lens and the protective sheath. The exemplary catheter can be placed near a center of the tube (e.g., 3.2 mm diameter), and the liquid-tube interface can correspond to the weak reflection at the bottom of the tomograms. It is easily seen that the speckle-decorrelation time (the speckle size in the horizontal direction) matches the expected relationship with particle speed. In particular, the horizontal speckle size gets bigger near the interfaces due to the no-slip condition of the flow.

For example, the speckle correlation time can be inversely proportional to the modulus of the velocity (the speed) of the particles

$$\tau \propto \frac{1}{|\vec{v}|} = \frac{1}{v}, \qquad (1)$$

The way of calculating the autocorrelation for speckle statistics can include, e.g., the use of the Pearson correlation function, either between A-lines at two consecutive times, or at a single or a group of depths as a function of time. This determination can produce an estimation of the decorrelation time that is highly influenced by noise, especially in the case of using the cross-correlation coefficient. Instead, according to an exemplary embodiment of the present disclosure, it is possible to utilize a modified correlation function tailored for the determination of speckle size, and analyze the speckle size at each depth as a function of time. The exemplary normalized correlation function can be defined as

$$g^{(2)}(\Delta t) = \frac{< \sum I(t)I(t + \Delta t) >}{\sum < I(t) >< I(t + \Delta t) >}, \qquad (2)$$

where <> denotes an ensemble average, *I* is the intensity, and the summation is along the discrete time dimension. In a system with unity speckle contrast and in absence of noise, this definition of the autocorrelation function has a maximum value of 2, totally decorrelated signals have a value of 1, and anti-correlated signals have values below 1. The ensemble average allows taking into account multiple correlation windows in the calculation, reducing the statistical fluctuations on speckle size and the effect of noise.

After certain testing, with the use of the above-defined autocorrelation function on the scattered radiation *amplitude,* as opposed to the traditional approach on the scattered radiation *intensity,* decorrelation profiles can be produced that can be significantly more homogeneous. This can be linked to the effect that the square has on outliers in the statistical fluctuations of speckle intensity. When this square is avoided, the outliers likely have a smaller weight on the autocorrelation function which produces more homogeneous results. A side effect of this can be that the autocorrelation no longer reaches a value of 2 for speckle with perfect contrast.

As noise is the most important factor in the accuracy of speckle decorrelation times, it is possible to define another exemplary autocorrelation by performing the following exemplary transformations: for example, it is possible to remove the value at $\Delta t = 0$, optionally apply a small Gaussian smoothing filter (FWHM = 3 px), renormalize the autocorrelation by defining the value at $\Delta t$ = 1 px as one, and define the median of the value in a certain range of the autocorrelation as zero

$$g^{(2)}(\Delta t) \equiv \frac{\tilde{g}^{(2)}_{\text{trad}}(\Delta t \geq 1\ \text{px}) - \text{median}\{\tilde{g}^{(2)}_{\text{trad}}(\Delta t > w)\}}{\tilde{g}^{(2)}_{\text{trad}}(\Delta t = 1\ \text{px}) - \text{median}\{\tilde{g}^{(2)}_{\text{trad}}(\Delta t > w)\}}, \qquad (3)$$

where $\tilde{g} \equiv g * c^{-\ln2\Delta t2/(\text{FWHM}/2)2}$ is the Gaussian filtered version, and $w$ is defined based on the autocorrelation properties. This corresponds to step 600 in the exemplary method shown in Figure 7. For example, by defining such exemplary autocorrelation, most if not all functions can have similar contrast and cover the range from 1 to approximately 0. The contrast $C$ is defined before the final normalization as

$$C = g^{(2)}_{\text{trad}}(\Delta t = 1\ \text{px}) - \text{median}\{g^{(2)}_{\text{trad}}(\Delta t > w)\}. \qquad (4)$$

$C$ can be a good indicator of presence of flow, similar to the speckle variance technique. This corresponds to step 610 in the exemplary method of Figure 7.

It may be preferable to estimate the noise floor of the exemplary system. This can be possible, e.g., if the system is fitted with a mechanism to block the sample arm of the interferometer while taking a measurement of scattered radiation. Then, a tomogram reconstruction on this data can be performed, and an average over all depths can provide an estimation for the noise floor. Further, most if not all scattered radiation measurements can be converted from intensity into SNR by subtracting the estimated noise floor value, corresponding to step 620 in the exemplary method shown in Figure 7.

In this exemplary embodiment, the decorrelation time for each region of the sample can be determined by calculating the time it takes the autocorrelation function calculated in step 600 to reach the value 0.5. This corresponds to step 630 in the exemplary method shown in Figure 7.

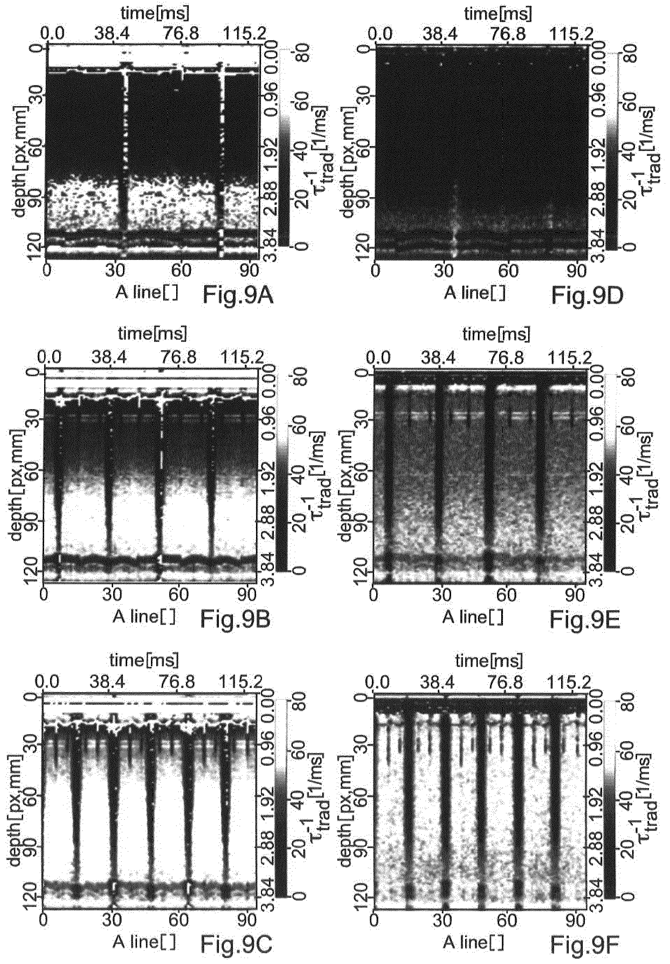

Figures 9A-9C show exemplary illustrations of complete inverse autocorrelation profiles for a solid material moving at a known and stable speed. The profiles were measured using the conventional autocorrelation method. The complete inverse autocorrelation profiles shown in Figures 9D-9F correspond to the same set of speeds and are measured by the exemplary embodiment of the speckle-amplitude statistics OFD1 system according to an exemplary embodiment of the present disclosure. Figures 9A-9F illustrate a significant improvement in the homogeneity of the profile using the exemplary embodiments of the present disclosure.

With respect to determining the speckle speed from the speckle decorrelation time (e.g., step 640 in the exemplary method shown in Figure 7), it is possible to consider a movement in the $x$ direction. In this exemplary case, the total apparent mean speed can be, e.g.:

$$\bar{v} = \sqrt{K^2_{BM} + v^2_x} + K_{BM}, \qquad (5)$$

where $K_{BM}$ denotes an offset given by stochastic movement, such as that produced by Brownian motion of the scatterers. The speckle-decorrelation time $\tau$ is inversely proportional to this speed. For example, zero speed may provide an infinite decorrelation time. However, because the autocorrelation can be calculated using a window of finite width, even at zero velocity, there may be a finite decorrelation time (equal to the window size). If necessary, an offset can be added to account for this effect to the $K_{BM}$ constant outside the radical to define a new offset $k_c$. Finally,

$$\tau^{-1} = k\sqrt{K^2_{BM} + v^2_x} + k_c \equiv \sqrt{k^2_{BM} + k^2 v^2_x} + k_c, \qquad (6)$$

where the $k$ proportionality constant has been absorbed into the new Brownian motion contribution constant.

Considering the exemplary embodiment shown in Figure 5 in which the scanning mechanism is a rotating catheter, the rotation of the catheter with angular velocity $\omega_R$ can produce decorrelation that is linked to motion in *the y* direction. However, there will not be a pure dependence on the tangential velocity $v_y = \omega_R z$ because as the catheter rotates, the light beam is not only displaced an amount $v_y dt$ but light is entering the material at a different angle $\theta(t + dt)$ = $\theta(t) + \omega_R dt$. As the speckle pattern is also dependent on incident angle, the combination of these exemplary movements can produce a speckle pattern whose size is mostly independent of $z$. It is possible to consider this exemplary decorrelation as arising directly from the angular velocity $\omega_R$ via another proportionality constant $k_R$, which can have some $z$ dependence. It is possible to see why the dependence is with the angular velocity of the catheter: if the whole sample were made to rotate at exactly the same speed, the decorrelation due to the rotation of the catheter would be exactly canceled.

Therefore, in a rotating catheter system with sample movement only in $x$, the speckle-decorrelation time can be described by, e.g.:

$$\tau^{-1} = \sqrt{k_{BM}^2 + k_R^2 \omega_R^2 + k^2 v_x^2} + k_c, \tag{7}$$

and the sample speed $|v_x|$ can be found using, e.g.:

$$|v_x| = \sqrt{\frac{(\tau^{-1} - k_c)^2 - k_{BM}^2 - k_R^2 \omega_R^2}{k^2}}, \tag{8}$$

where it is made explicit that it is not possible to determine the sign of the flow velocity. This corresponds to step 640 in the exemplary method of Figure 7 when sample motion happens only in the $x$ direction.

Considering, e.g., the corrected decorrelation time $\tau_c^{-1} \equiv \tau^{-1} - k_c$, the equation above indicates that at high flow speeds, the rotation between $\tau_c^{-1}$ and speed is linear

$$\lim_{|v_x| \to \infty} \tau_c^{-1} = k|v_x|, \tag{9}$$

while it can be non-linear at low flow speeds with an offset at zero flow speed given by Brownian motion and catheter rotation

$$\lim_{|v_x| \to 0} \tau_c^{-1} = \sqrt{k_{BM}^2 + k_R^2 \omega_R^2}. \tag{10}$$

This indicates that speckle-decorrelation flow measurements can be better suited for quantifying high flow speeds than Doppler, while the opposite can be the case when the rotational speed of the catheter is significant.

In the general case of sample motion in any direction, the proportionality constants can be different if the voxel that corresponds to the point spread function (PSF) is asymmetric. This can be usually the case, unless some post-processing on the OFDI data is performed. This exemplary case is described by, e.g.:

$$\tau^{-1} = \sqrt{k_{BM}^2 + k_R^2 \omega_R^2 + k_{pp}^2 v_x^2 + k_{pp}^2 v_y^2 + k_{pl}^2 v_z^2} + k_c, \tag{11}$$

where $k_{pp}$ is the proportionality constant for flow perpendicular to the beam, $k_{pl}$ the constant for flow parallel to the beam, $v_y$ is the tangential speed of the flow in the $y$ direction, and $k_R^2 \tilde{\omega}_R^2$ is a term that represents the decorrelation due to the angular scanning, and can be dependent on depth and tangential speed. To understand the effect of the two constants that depend on the sample velocity, it is possible to assume for simplicity no motion in the $y$ direction. Therefore

$$|\hat{v}_{xz}| \equiv \sqrt{\frac{k_{pp}}{k_{pl}} v_x^2 + \frac{k_{pl}}{k_{pp}} v_z^2} = \sqrt{\frac{(\tau^{-1} - k_c)^2 - k_{BM}^2 - k_R^2 \omega_R^2}{k^2}}, \tag{12}$$

where it is clear that $|\hat{v}_{xz}| \neq |v_{xz}| \equiv \sqrt{v_x^2 + v_z^2}$ due to the different constants. In order to avoid this it is necessary to rescale the tomogram in the axial direction to make the axial and transversal PSFs of the system equal. If the tomogram is rescaled to have a symmetric PSF and define now $k \equiv k_{pp} = k_{pl}$ in the case of no flow in the $y$ direction, e.g.:

$$|v_{xz}| = \sqrt{v_x^2 + v_z^2} = \sqrt{\frac{(\tau^{-1} - k_c)^2 - k_{BM}^2 - k_R^2\omega_R^2}{k^2}}. \qquad (13)$$

The case of flow in the $y$ direction can be more complex because of the coupling with the rotational contribution. However, many exemplary methods for determining the total speed can be utilize, such as, e.g., measuring at different rotational speeds solving the equations for $\upsilon^2$.

Further exemplary embodiments can be provided with a usage of a signal-to-noise (SNR) ration calibration method. The rationale for the parametrization can be the following: when measuring a solid sample with a static catheter $k_{BM} = \omega_R = 0$, so $\tau^1$ as a function of speed should be a linear function, although when reaching $\tau_{\max}^{-1}$ the slope should increase to indicate that higher speeds will all produce values close to the maximum inverse correlation time given by the time resolution of the system. The exemplary illustrations in Figure 9 were used as calibration data to parametrize sample speed as a function of inverse correlation time and SNR. The best parametrization for this behavior was a fourth order polynomial where the linear term dominates except for correlation times near the limit of the system. The exemplary behavior of the SNR. can be mostly linear for > 25 dB SNR and higher-order for lower SNR.

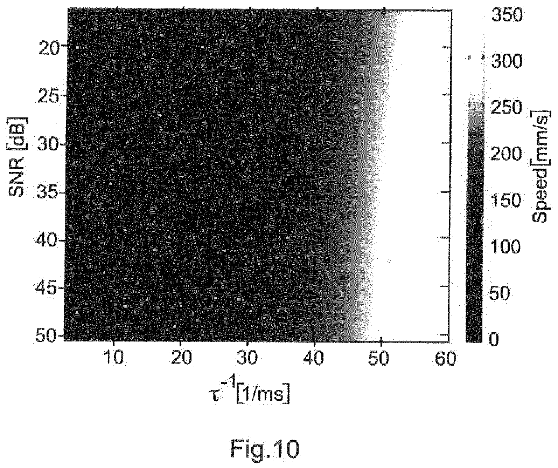

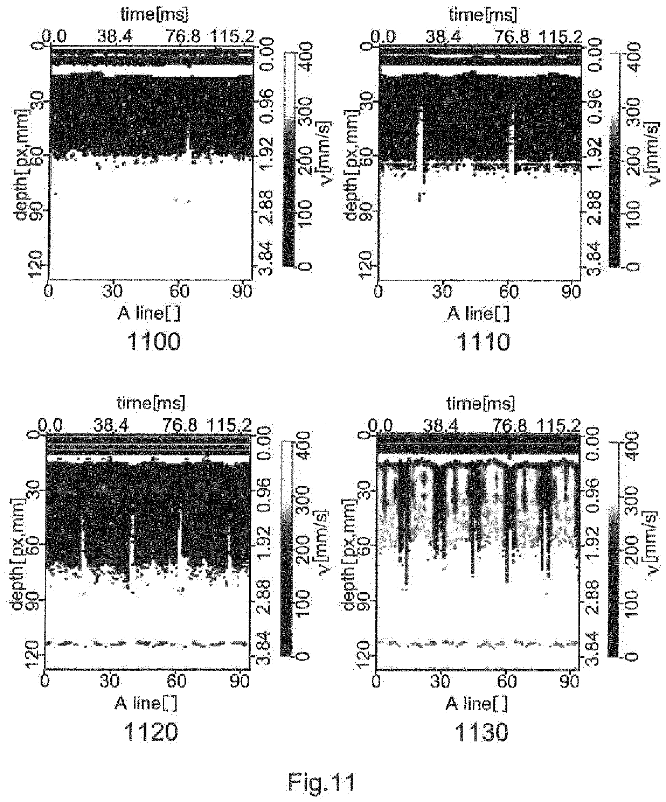

Therefore, the speed was parametrized as a fourth order polynomial of the two variables $\tau^1$ and SNR and a fit of this exemplary model to the data can be performed by minimizing the mean absolute value error, instead of the mean quadratic error, to minimize the influence of outliers. An exemplary parametrization based on the present exemplary material as measured by the exemplary embodiment of the speckle-amplitude statistics OFDI system according to an exemplary embodiment of the present disclosure is shown in an exemplary illustration of Figure 10. Figure 11 shows a set of an exemplary illustrations of speed profiles for a moving solid phantom as measured using the exemplary parametrization of speed as a function of $\tau^1$ and SNR based on the present exemplary material as measured by the exemplary embodiment of the speckle-amplitude statistics OFDI system according to an exemplary embodiment of the present disclosure. The solid phantom was translated by using a motorized linear stage at a fixed speed, corresponding to 80 mm/s (illustration 1100), 100 mm/s (illustration 1110), 200 mm/s (illustration 1120) and 300 mm/s (illustration 1130). The measured speed by our technique matches extremely well the expected values.

Another exemplary calibration relates the newly defined inverse correlation time with the velocity of a given material. As a matter of illustration, the scattering liquid used in the exemplary configuration can be intralipid and the results from this fitting procedure are:

$$\begin{aligned} k_{BM}^2 &= 734.9 \\ k^2 &= 0.177 \\ k_c &= -16.1 \end{aligned} \qquad (14)$$

## EXAMPLES OF MULTIDIMENSIONAL SPEED PROFILES

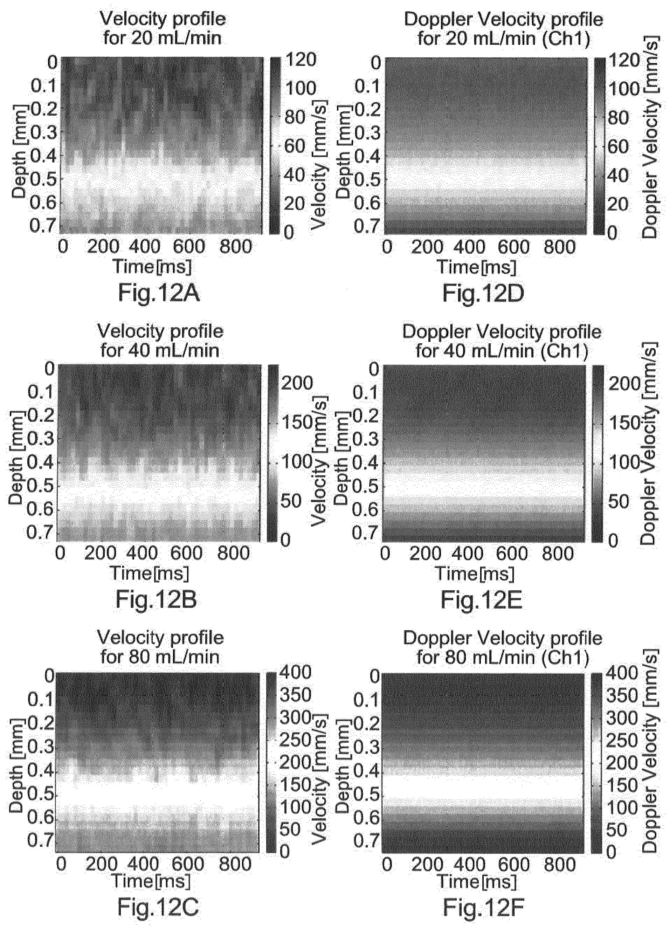

The system and methods described above allows for the accurate measurement of speeds from speckle-amplitude statistics as described in the present disclosure. Figures 12A-12F shows illustrations of measurements taken of Intralipid flow through a tube at different flow rates when the catheter is not rotating. In particular, Figures 12A-12C provide exemplary illustrations of speed profiles for a flowing liquid as measured by the exemplary embodiment of the speckle-amplitude statistics OFDI system according to an exemplary embodiment of the present disclosure. Further, Figures 12D-12F show exemplary illustrations of the flow speed as measured by Doppler analysis using the exemplary embodiment of the phase-correction OFDI system according to an exemplary embodiment of the present disclosure.

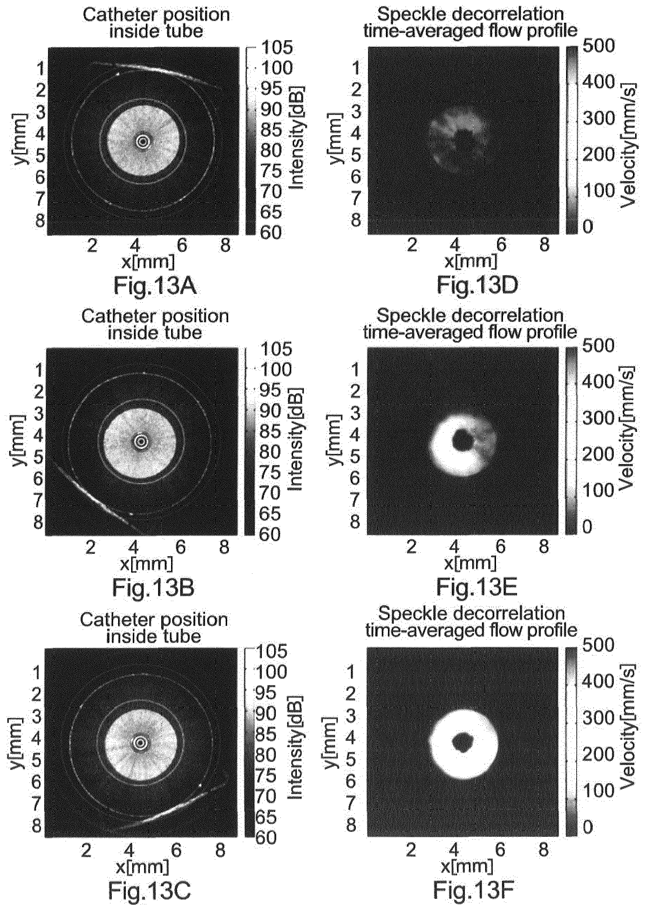

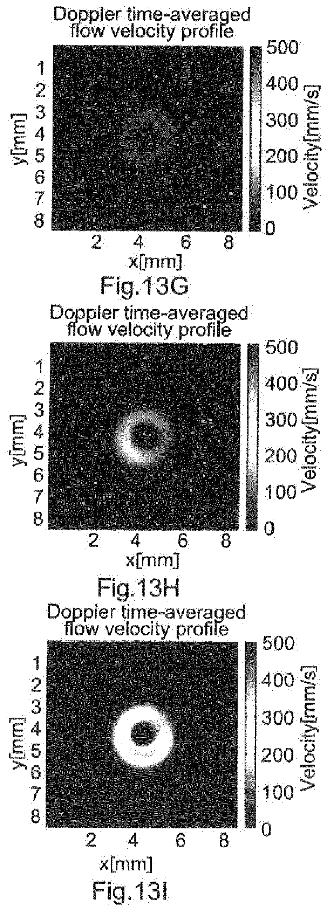

Figure 13 shows a set of illustrations of exemplary two-dimensional speed profile measurements when the catheter is rotating where the contribution from rotation has been calibrated as

$$\begin{aligned} k_{BM}^2 + k_R^2\omega_R &= 7310 \\ k^2 &= 0.156 \\ k_c &= -43.0 \end{aligned} \qquad (15)$$

In particular, Figures 13A-13C show exemplary illustrations of the conventional structural image from OFDI of the tube and the liquid. Figures 13D-13F show exemplary illustrations of two-dimensional speed profiles for a flowing liquid as measured by the exemplary embodiment of the speckle-amplitude statistics OFDI system and method according to an exemplary embodiment of the present disclosure. Further, Figures I3G-13I show exemplary illustrations of two-dimensional flow speeds as measured by Doppler analysis using the exemplary embodiment of the phase-correction OFDI system and method according to an exemplary embodiment of the present disclosure

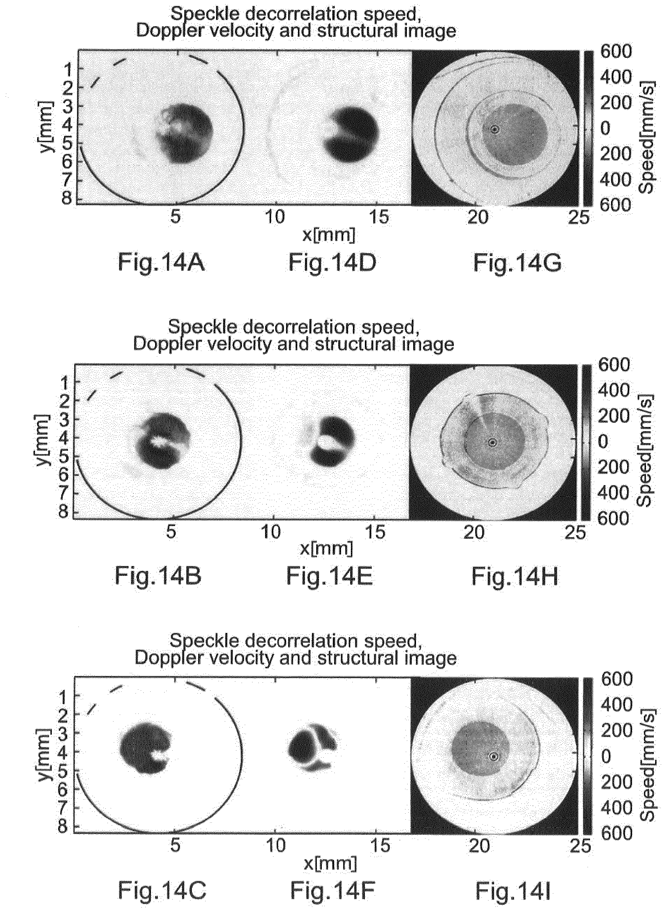

The system according to an exemplary embodiment of the present disclosure can be further configured to provide an additional scanning mechanism in 300m in Figure 5. For example, a pullback mechanism can provide longitudinal scanning of the sample 320. If the measurements described above can be carried out while the pullback system is in operation, it is possible to obtain three-dimensional speed profiles of the sample. Figures 14A-14I provide a set of illustrations of three-dimensional speed profile measurements when the catheter is rotating using the above-described exemplary embodiment of the systems and methods according to the present disclosure. In particular, Figures 14A-14C show exemplary illustrations of three-dimensional speed profiles for a flowing liquid at different planes in the tube longitudinal direction as measured by the exemplary embodiment of the speckle-amplitude statistics OFDI system and method according to an exemplary embodiment of the present disclosure. In addition, Figures 14D-14F show exemplary illustrations of three-dimensional speed profiles for a flow velocity measured by Doppler analysis using the exemplary embodiment of the phase-correction OFDI system and method according to an exemplary embodiment of the present disclosure. Further, Figures 14G-14I show exemplary illustrations of conventional structural images from OFDI of the tube and the liquid.

## MULTIDIMENSIONAL VECTORIAL SPEED AND VELOCITY PROFILES

For example, the scanning speed can contribute to the decorrelation time. The equivalent in a tabletop scanning system can correspond to a translational speed, which can have a simpler relationship with decorrelation, In this exemplary case, the angle of incidence does not change, although there can be a scanning translational speed in the $xy$ plane. If the scanning speeds are much lower than sample speeds, this correction can be small and can be ignored. If they are, or are made comparable it is possible to make use of different scanning speeds to determine the individual components of the velocity vector in the transversal plane. For example, by scanning in the $y$ direction with velocity $v_s$ we have

$$\frac{(\tau^{-1} - k_c)^2 - k_{BM}^2 - k^2 v_s^2}{k^2} = v_x^2 + v_y^2 + v_z^2 \pm v_s v_y = v^2 \pm v_s v_y. \qquad (16)$$

By measuring with different scanning speeds in $x$ and $y$, several of these equations can be obtained and solved for $v^2$, $v_x$, $v_y$ and $v_z$.

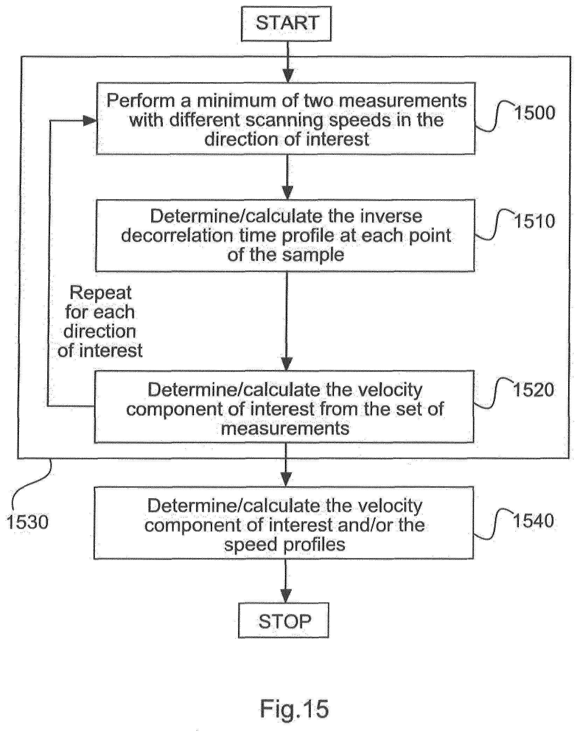

Following the reasoning of Eq.(16), an exemplary embodiment of a method according to the present disclosure as shown in Figure 15 can be provided as follows:

1. Perform a minimum of two measurements with different scanning speeds in the direction in which the velocity component $v_a$ that is to be determined. In the case of a translational scanning system this corresponds to at least two scanning speeds $v_s$ (step 1500).

2. Analyze the speckle decorrelation according to exemplary embodiment of Figure 7. The resulting decorrelation time is described by $(\tau^{-1} - k_c)^2 = k_{BM}^2 + k^2 v^2 \pm k^2 v_s v_a + k^2 v_s^2$ (step 1510).

3. Because all constants and $v_s$ are known, from these two or more equations determine $v_a$ (step 1520).

4. Repeat steps 1, 2 and 3 for all directions of interest (step 1530).

5. If at least two directions of interest are measured, the equations can be solved for $v^2$, $v_x$ and $v_y$, and $|v_z|$ can be extracted from $|v_z| = v^2 - \sqrt{v_x^2 + v_y^2}$ (step 1540).

In a further exemplary embodiment, it is possible to extract two- and three-component vectorial velocity profiles of the sample, depending on the scanning/imaging implementation. In the case of a mechanically scanning system in which time and the transversal coordinates are coupled a two-component vectorial profile can be determined, where the two components correspond to the longitudinal speed and the transversal speed. Eq.(11) can be written as

$$\tau^{-1} = \sqrt{k_{BM}^2 + k_R^2 \omega_R^2 + k_{pp}^2 v_{pp}^2 + k_{pl}^2 v_{pl}^2} + k_c, \qquad (17)$$

where the transversal speed is $v_{pp}^2 = v_x^2 + v_y^2$ and the longitudinal speed is

$$v_{pl}^2 = v_z^2.$$

$k_{pl}$ can be synthetically modified by different methods after measuring, because its value depends on the axial resolution of the tomogram. For different axial resolutions, its value can be calibrated. One possibility includes splitting the raw spectrum and reconstructing different realizations of the tomogram with a reduced bandwidth, which results in a tomogram with reduced longitudinal resolution. In general, it is possible to consider such exemplary process as signal filtering in k-space, and the use of several filters in k-space can be considered as k-space filtering diversity. Multiple possibilities of k-space filtering are possible, which can provide numerous diversities, such as, e.g., axial resolution diversity, group velocity dispersion diversity, quartic dispersion diversity, etc. For axial and lateral flow discrimination, we will focus on axial resolution diversity. This exemplary step/procedure does not require a phase stability, as all A-lines can be inherently phase-stable with respect to points inside the same A-line. Exemplary different realizations can be used in the calculation of the autocorrelation function (step 600 in Figure 7) to improve the SNR. As each realization will be described by an Eq.(17) with a different $k_{pl}$, a system of equations can be created and solved for $v_{pl}^2$ and $v_{pp}^2$. In general, modifications to the k-space signal can produce a different $k_{pl}$, which can then be used to provide an exemplary system, method and/or computer-accessible medium which can be configured and/or programmed to utilize equations, and solve for axial and lateral speeds.

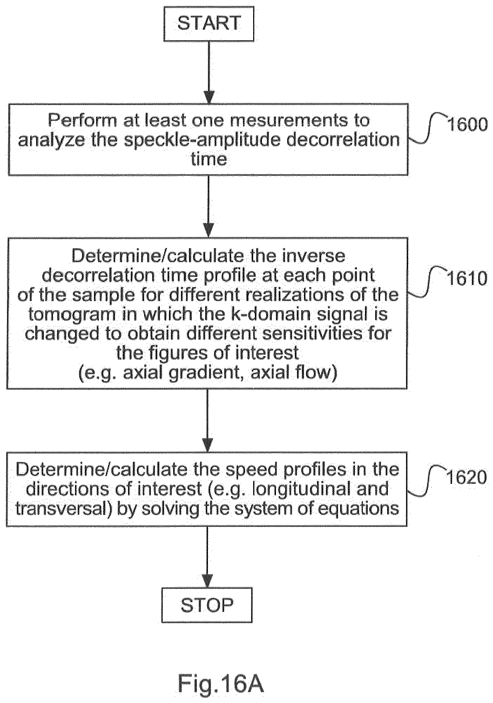

Following the reasoning of Eq.(17), an exemplary embodiment of a method according to the present disclosure as shown in Figure 16A, can be provided as follows:

6. Perform at least one measurement to analyze the speckle-amplitude decorrelation time (step 1600').

7. Analyze the speckle decorrelation according to exemplary embodiment of Figure 7. The resulting decorrelation time is described by $\tau^{-1} = \sqrt{k_{BM}^2 + k_R^2 \omega_R^2 + k_{pp}^2 v_{pp}^2 + k_{pl}^2 v_{pl}^2 + k_c}$, (step 1610').

8. Create or provide a different realization of the tomogram by, e.g., modifying the signal in k-space. For example, reduce the bandwidth in order to have smaller $k_{pt}$ constant for this realization. The speckle decorrelation of this realization can be analyzed (continuation of step 1610').

9. Provide an exemplary system, method and/or computer-accessible medium which can be configured and/or programmed to utilize equations from the result for performing each analysis, and considering that the different $k_{pl}$ constants are known via a calibration, solve for $v_{pl}^2$ and $v_{pp}^2$ (step 1620').

There can be other figures of interest that can be determined in a similar manner, e.g., by k-space filtering. In general, interferometric systems can have varying degrees of dispersion mismatch between the reference and sample arms. Furthermore, it is possible to add or subtract the dispersion mismatch synthetically after measuring. As a particular exemplary embodiment, consider the case of so-called group velocity dispersion (GVD). A variation in GVD influences the decorrelation produced by axial velocity gradients, which can then be used to determine them. Consider that the system has a GVD given by a quadratic dispersion of amplitude $2\pi\gamma$. This can be produced either by, e.g., physical GVD between the two arms of the interferometer, and/or synthetically in post processing (e.g., k-space filtering producing GVD diversity). In presence of GVD, the complex amplitude spread function (ASF) of the system is

$$ASF(k) = \sqrt{\frac{w_z}{\hat{w}_z}} \exp\left(-2ik_0 z\right) \exp\left(-z^2/\hat{w}_z\right) \exp\left(-i\pi\gamma z^2/\hat{w}_z\right), \qquad (18)$$

where z is the axial direction, $k_0$ the central wave number of the spectrum, $w_z$ the diffraction-limited axial 1/e size of the point spread function (PSF), and $\hat{w}_z$ the actual 1/e size of the PSF due to the quadratic dispersion.

Assuming an axial speed profile inside the ASF with a linear gradient of the form $v_z(z) = v_{z0} + z v_{z1} / w_z$. After some approximations, the autocorrelation function becomes

$$(19)$$

$$g^{(2)}(\tau) = 1 + |g^{(1)}(\tau)|^2 \approx 1 + \exp\left(-8n^2 k_o^2 D\tau\right) \exp\left(-v_z^2 \tau^2/w_z^2\right) \exp\left(-2v_{xy}^2 \tau^2/w_{xy}^2\right) \times$$

$$\times \exp\left(-\frac{1}{2}\ln\left(\frac{25}{4} + \pi^2 \gamma^2 \frac{9}{4}\right) v_{z1}\tau/w_z\right) \exp\left(-2\pi\gamma k_0 v_{z0} v_{z1}\tau^2/w_z^2\right) \exp\left(-k_0^2 v_{z1}^2 \left(1 + \pi^2\gamma^2\right)\tau^2\right),$$

where $xy$ are the transverse directions, $n$ is the refraction index, $D$ the diffusion constant of the scatterers, and $w_{xy}$ the lateral $1/e$ size of the PSF. By defining the speckle decorrelation time $\tau_c$ as the time when the second-order autocorrelation function reaches the threshold $1 + g_c$, the following is provided:

$$(20)$$

$$\tau_c^{-1} = K_{BM} + GVD_1(\gamma) + \sqrt{[K_{BM} + GVD_1(\gamma)]^2 + GVD_2(\gamma)^2 + \hat{g}_c v_z^2/w_z^2 + 2\hat{g}_c v_{xy}^2/w_{xy}^2 + k_0^2 v_{z1}^2},$$

where the diffusion constant is $K_{BM} = 4\hat{g}_c n^2 k_o^2 D$, the group velocity dispersion contributions are

$$GVD_1(\gamma) = \frac{\hat{g}_c}{2}\ln\left(\frac{25}{4} + \pi^2\gamma^2\frac{9}{4}\right)\frac{v_{z1}}{w_z}$$

and $GVD_2(\gamma) = 2\pi\gamma k_0 v_{z0} v_{z1}/w_z^2 + k_0^2 v_{z1}^2 \pi^2\gamma^2$. We identify the axial velocity gradient contribution to decorrelation as the last term in Eq. (20). It is easy to see that by varying $\gamma$ synthetically, we can find the decorrelation contribution from $v_{z1}$ by producing different values of the decorrelation time due to $GVD_1(\gamma)$ and $GVD_2(\gamma)$. The data can be assembled in an exemplary system, method and/or computer-accessible medium which can be configured and/or programmed to utilize equations to find the axial velocity gradient contribution to decorrelation. Further, e.g., when physical dispersion mismatch is present, its effects persist even when compensated synthetically. This can be found by performing an exemplary calibration when the axial velocity gradient is known, which produces the $GVD_1(\gamma)$ and $GVD_2(\gamma)$ baseline contributions from the physical dispersion mismatch.

Following the reasoning of Eq.(20), an exemplary embodiment of a method according to the present disclosure as shown in Figure 16A can be provided as follows:

10. Perform at least one measurement to analyze the speckle-amplitude decorrelation time (step 1600).

11. Analyze the speckle decorrelation according to exemplary embodiment of Figure 7. The resulting decorrelation time is described by

$$\tau_c^{-1} = K_{BM} + GVD_1(\gamma) + \sqrt{[K_{BM} + GVD_1(\gamma)]^2 + GVD_2(\gamma)^2 + \hat{g}_c v_z^2/w_z^2 + 2\hat{g}_c v_{xy}^2/w_{xy}^2 + k_0^2 v_{z1}^2},$$

with $GVD_1(\gamma) = \frac{\hat{g}_c}{2}\ln\left(\frac{25}{4} + \pi^2\gamma^2\frac{9}{4}\right)\frac{v_{z1}}{w_z}$ and $GVD_2(\gamma) = 2\pi\gamma k_0 v_{z0} v_{z1}/w_z^2 + k_0^2 v_{z1}^2 \pi^2\gamma^2$ (step 1610).

12. Create a different realization of the tomogram by filtering the signal in k-space. For example, add GVD by adding to the signal a quadratic phase for a new value of $\gamma$. Analyze the speckle decorrelation of this realization (step 1610).

13. Provide an exemplary system, method and/or computer-accessible medium which can be configured and/or programmed to utilize equations from the result for each analysis, and considering that the $GVD_1$ and $GVD_2$ contributions are different for each $\gamma$, solve for $k_0^2 v_{z1}^2$. If there is a physical dispersion baseline contribution, this would add $GVD_1$ and $GVD_2$ contributions that can be found in a calibration (step 1620).

In the case of an imaging system (in which the tomograms of transversal one- or two-dimensional regions are taken simultaneously, for example using a camera in which each pixel corresponds to different transversal positions of the sample), the A-lines taken simultaneously can be phase-coherent. For this reason, it is possible to synthetically alter the transversal resolution in one of the two transverse dimensions (for example, by calculating the convolution of the two- or three-dimensional complex-valued tomogram with a kernel in one of the transverse directions), and generate a number of realizations of the tomogram which can be described by an equation similar to Eq.(17) where the proportionality constant that changes is the $k_{pp}$ in the transversal direction where the filter is applied. It is possible to determine a three-dimensional profile if the above-explained technique is performed first, so the values for $v_{pl}^2$ and $v_{pp}^2$ are already known. Then ,using the exemplary technique for transversal manipulation of the resolution, an exemplary embodiment of the system of equations can be provided and can be used solve for $v_x^2$ or $v_y^2$ (depending on which one has the filter applied) and given that $v_{pp}^2 = v_x^2 + v_y^2$ is known, solve for the remaining transversal component.

Following the reasoning above, a method according to another exemplary embodiment of the present disclosure shown in Figure 16A can be provided as follows;

 1. Perform at least one measurement to analyze the speckle-amplitude decorrelation time (step 1600').
 2. Synthetically modify the signal in k-space, such as reducing the bandwidth to reduce the resolution in the direction of interest (e.g. axial) and perform a decorrelation time analysis. Prior to the decorrelation time analysis, optionally perform a correction for axial velocity gradient effects using GVD as explained above. In the case of a rotational-scanning system in which the axial resolution is varied each reconstruction is described by

$$\tau^{-2} - k_{BM}^2 - k_R^2 \omega_R^2 - k_c = k_{pp}^2 v_{pp}^2 + k_{pl}^2 v_{pl}^2$$ , where only $k_{pl}$ depends on the axial resolution (step 1610').

 3. Use the system of equations generated in the previous step (step 1610) with an exemplary system, method and/or computer-accessible medium which can be configured and/or programmed to solve for the square of the velocity component of interest (e.g. $v_{pp}^2$ and $v_{pl}^2$ in the case of axial resolution change) (step 1620).

In another exemplary embodiment, it is possible to extract two-component vectorial velocity profiles of the sample, where the two components correspond to the longitudinal speed and the transversal speed using amplitude-based and phase-based velocity measurements. Speckle amplitude-based analysis provides information about the total speed of the sample $v$, while phase-based Doppler analysis provides $v_z = v_{pl}$, which can then be used to determine the transversal speed profile

$$v_{pp} = \sqrt{v^2 - v_{pl}^2}.$$

Following the reasoning above, a method according to an exemplary embodiment of the present disclosure shown in Figure 17 can be provided as follows:

 1. Perform at least one measurement to analyze the speckle-amplitude decorrelation time and phase-based Doppler (step 1700).
 2. Provide an exemplary system, method and/or computer-accessible medium which can be configured and/or programmed to utilize equations using the results from both techniques, and solve for $v_{pp}^2$ and $v_{pl}^2$ (step 1710).

**EXAMPLES OF AXIAL VELOCITY GRADIENT CORRECTION AND AXIAL AND LATERAL SPEED PROFILES**

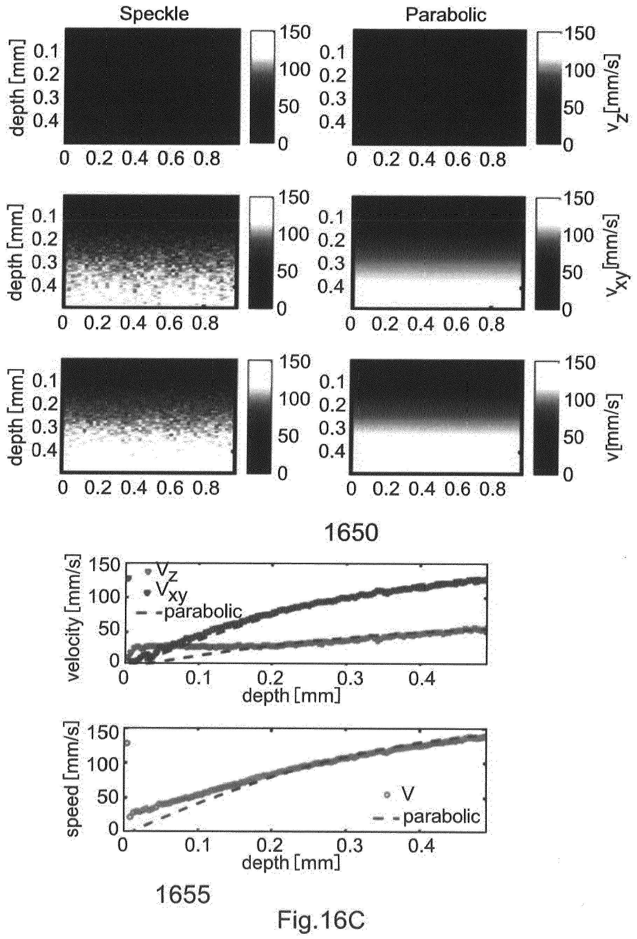

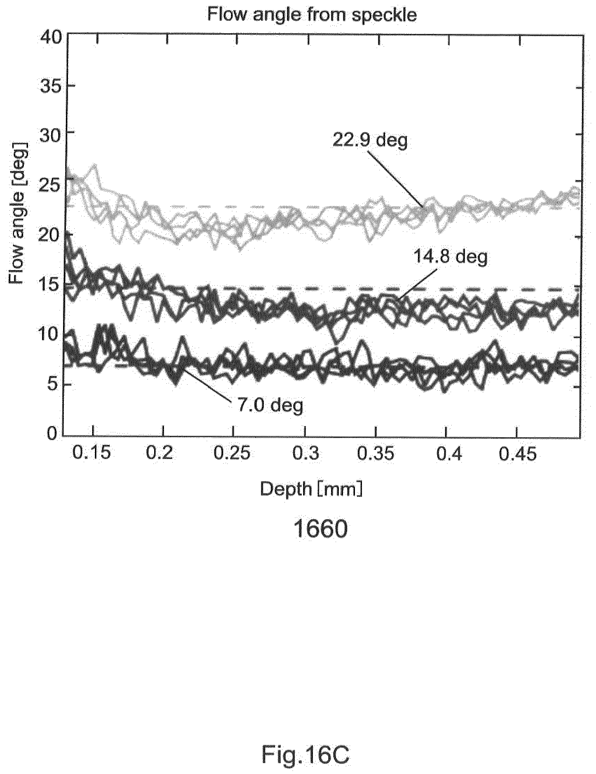

The exemplary systems, methods, techniques and computer-accessible medium described herein above facilitates an accurate measurement of speeds from speckle-amplitude statistics in presence of axial velocity gradients, as well as the determination of the axial and lateral components as described in the present disclosure. Figure 16C shows a set of illustrations of measurements taken of Intralipid flow through a tube at different flow rates to correct for axial velocity gradient errors in speckle decorrelation speed measurements as measured by the exemplary embodiment of the speckle-amplitude statistics OFDI system and method according to the present disclosure.

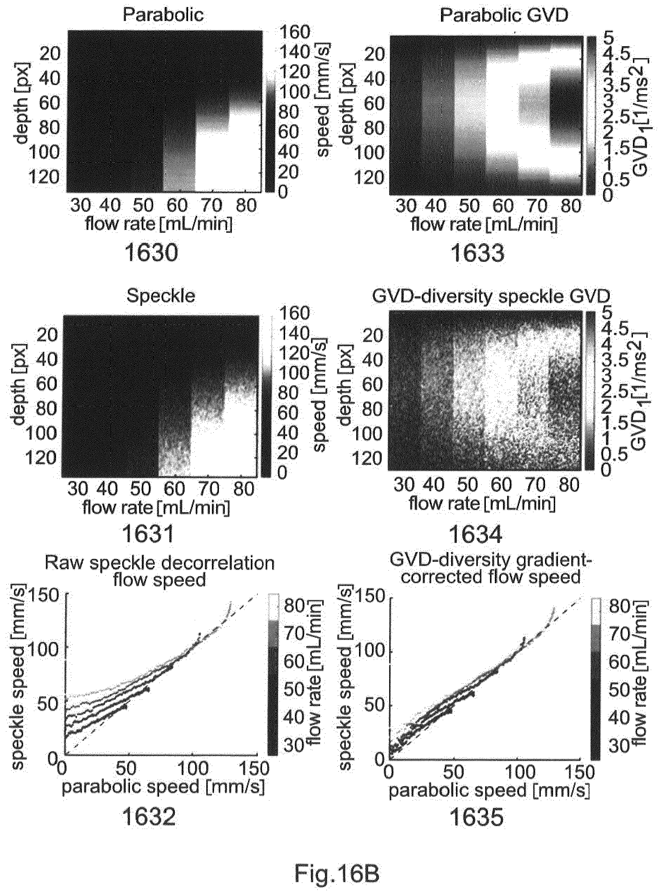

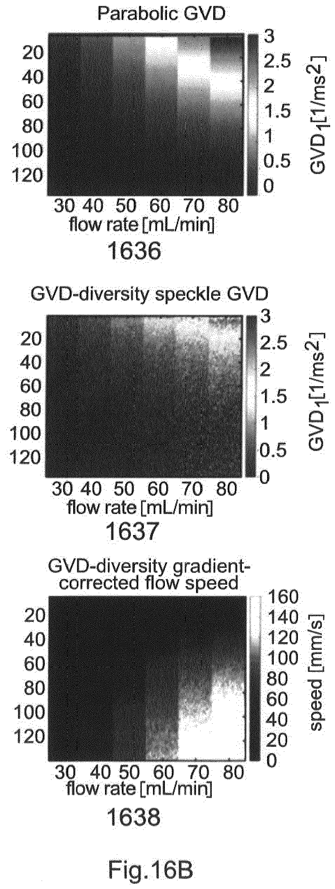

For example, image 1631 of Figure 16B are exemplary illustrations of axial velocity gradient determination using the exemplary embodiment of the k-space GVD diversity analysis for a flowing liquid as measured by the exemplary embodiment of the speckle-amplitude statistics OFDI system and method according to the present disclosure. Indeed, the exemplary image 1630 is the expected parabolic speed profile, the exemplary image 1631 is the uncorrected speckle flow speed profile, the exemplary image 1632 is the rotation between data in images 1630 and 1631, the exemplary image 1633 is the expected GVD_1 due to axial velocity gradient, 1634 is the GVD_1 as determined by the methods according to the exemplary embodiments shown in Figures 16A (1600, 1610, 1620), the exemplary image 1636 is the expected GVD_2 due to axial velocity gradient, the exemplary image 1637 is the GVD_2 as determined by the methods according to the exemplary embodiments of Figures 16A (1600, 1610, 1620), the exemplary image 1638 is the corrected speckle flow speed profile, and the exemplary image 1635 is the relation between data in the images 1630 and 1638.

Figure 16C shows a set of illustrations of exemplary speed profile measurements of axial, lateral and total flow speed, in which the axial gradient velocity correction was used for more accurate results, e.g., of the k-space bandwidth diversity analysis for a flowing liquid as measured by the exemplary embodiment of the speckle-amplitude statistics OFDI system and method according to the present disclosure. In particular, the exemplary set of images 1650 shows on the left column thereof the speckle determined flow, on the right column the parabolic expected flow, the top row - the axial flow, in the center row - the lateral flow, and at the bottom row - the total flow speed, e.g., as determined by the method according to the exemplary embodiments of Figures (see exemplary procedures/steps 1600, 1610, 1620). The exemplary set of images 1655 indicate average profiles and expected profiles shown in the exemplary images of 1650. The exemplary image 1660 shows the corresponding flow angle as a function of depth as determined for 3 different angles in the region of interest using the exemplary embodiment of lateral and axial flow determination according to an exemplary embodiment of the present disclosure.

## EXEMPLARY TURBULENCE DETERMINATION IN FLOW SPEED PROFILES

There can be different approaches for determining turbulence when the speed profile corresponds to a flowing material. The method shown as a flow diagram in Figure 18 illustrates the invention defined in the appended claims. The steps/procedures shown in Figure 18 are as follows:

1. Determine the Doppler velocity profile in the LOS, step 1800.
2. Determine the total speed profile using speckle decorrelation, step 1810.
3. Laminar flow inside a cavity usually exists only when the direction of the flow is homogeneous, therefore the absolute value of the Doppler velocity profile is proportional to the actual speed profile. Subtracting the Doppler profile from the speckle profile will highlight areas where the two profiles disagree, which directly mark the areas with flow with different directions, step 1820.
4. Areas with large discrepancies are marked as areas with turbulent flow to avoid false positives due to statistical fluctuations of the speckle speed profile, step 1830

For example, by implementing a method for determining a vectorial velocity profile based on speckle decorrelation (such as the exemplary method shown in Figure 16), no assumptions on the direction of the flow inside a cavity are needed in order to detect turbulent flow. The vectorial profile can provide a directional information, and analysis of this information (such as a velocity direction variance profile) can provide direct information on turbulence.

## EXEMPLARY TRACKING OF MOTION OF PROBE

For example, by measuring the relative motion of the probe and sample, instead of determining the velocity profile of the sample, if the sample corresponds to a monolithic structure, this information can be used to track the motion of the probe during measurements. In the exemplary embodiment of the system shown in Figure 5, although the blood flow inside the vessel carries information of interest, the vessel wall is a monolithic landmark that can be used to track the movement of the catheter while it is performing measurements. This can be important when performing a pullback scan (scanning in the longitudinal direction of the vessel), as unwanted motion of the tissue can lead to structural imaging and flow imaging artifacts. The exemplary embodiment of the method according to the present disclosure can be used to track the motion of the probe without the need of additional radiation apart from the configuration and/or technique used for imaging. It is also possible to track not only displacements, but also the rotational speed of the catheter as it scans the tissue. Irregular rotational scanning, known in the literature as non-uniform rotation distortion (NURD), is a prevalent problem in catheter-based imaging. The exemplary embodiment of the method according to the present disclosure can track the rotational speed of the catheter to compensate for such distortions.

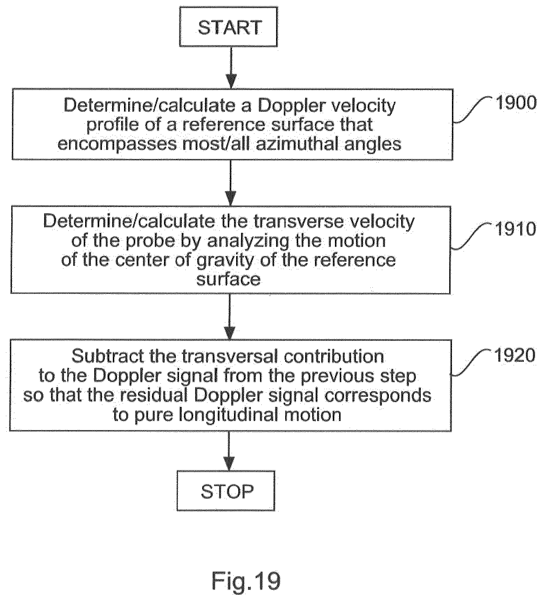

Another exemplary embodiment of a method according the present disclosure is shown in Figure 19, and provided as follows:

1. Determine/calculate a Doppler velocity profile of a reference surface. The surface should ideally encompass all or most azimuthal angles, such as the vessel in Figure 5, It can be readily recognized that many reference surfaces are possible depending on the application, such as the wall of the airways in pulmonary applications, or the wall of the esophagus in gastrointestinal applications (step 1900).

2. Determine/calculate the transversal velocity of the probe by analyzing the motion of the center of gravity of the reference surface. This is accomplished by a proper model of the reference surface (e.g. an ellipsoid in the case of a blood vessel), and implementation of computer vision algorithms to track the wall (step 1910).

3. Subtract the transversal contribution to the Doppler signal from the previous step so that the residual Doppler signal corresponds to pure longitudinal motion. As the Doppler signal has contribution from both longitudinal and transversal motion (due to the angle β in Figure 5), the transverse contribution has to be determined/calculated from the data from step 1910 and then subtracted from the experimental Doppler data. This provides a Doppler signal that comes exclusively from longitudinal motion of the catheter (step 1920).

Using the information from the longitudinal motion of the catheter and the transversal motion derived from image analysis, e.g., the motion of the catheter in three-dimensional space can be obtained. This can be used, for instance, to correct image artifacts from inhomogeneous pullback speeds, unwanted vessel motion, patient motion, among other applications.

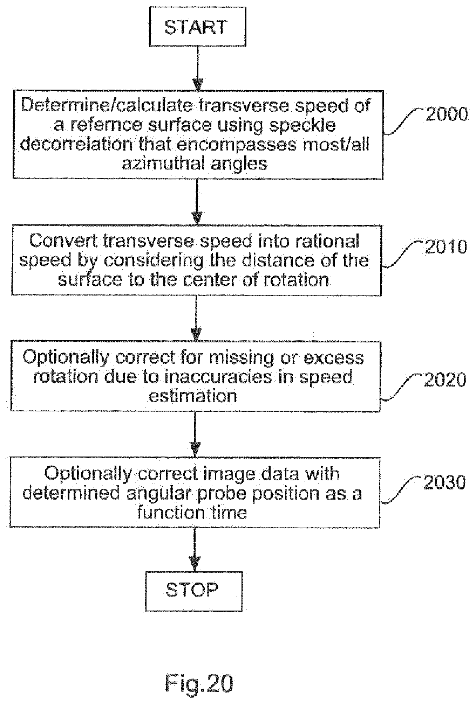

Another exemplary embodiment of a method according to another exemplary embodiment of the present disclosure is shown in Figure 20, and provided as follows:

1. Determine/calculate the transverse speed of the probe by analyzing the speckle decorrelation of a reference surface. This surface should ideally encompass all or most azimuthal angles, such as the vessel in Figure 5. It can be readily recognized that many reference surfaces are possible depending on the application, such as the wall of the airways in pulmonary applications, or the wall of the esophagus in gastrointestinal applications (step 2000).

2. Convert transverse speed into rotational speed by taking into account the distance of the surface to the center of rotation. Use transverse speed to calculate angular position as a function of time (step 2010).

3. Optionally correct for missing or excess speed due to inaccuracies in the speed estimation (step 2020).

4. Optionally correct the image data taking into account the determined angular position of the probe as a function of time (step 2030).

## EXAMPLES OF LONGITUDINAL AND ROTATIONAL PROBE MOTION TRACKING

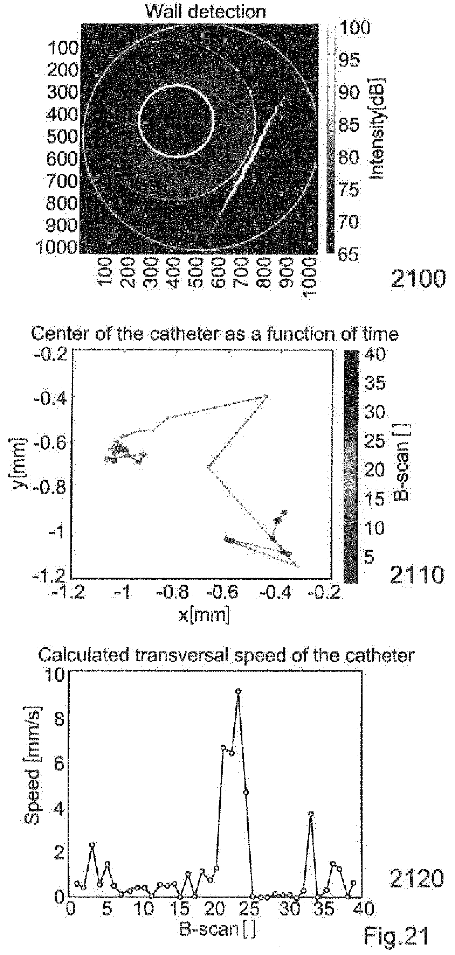

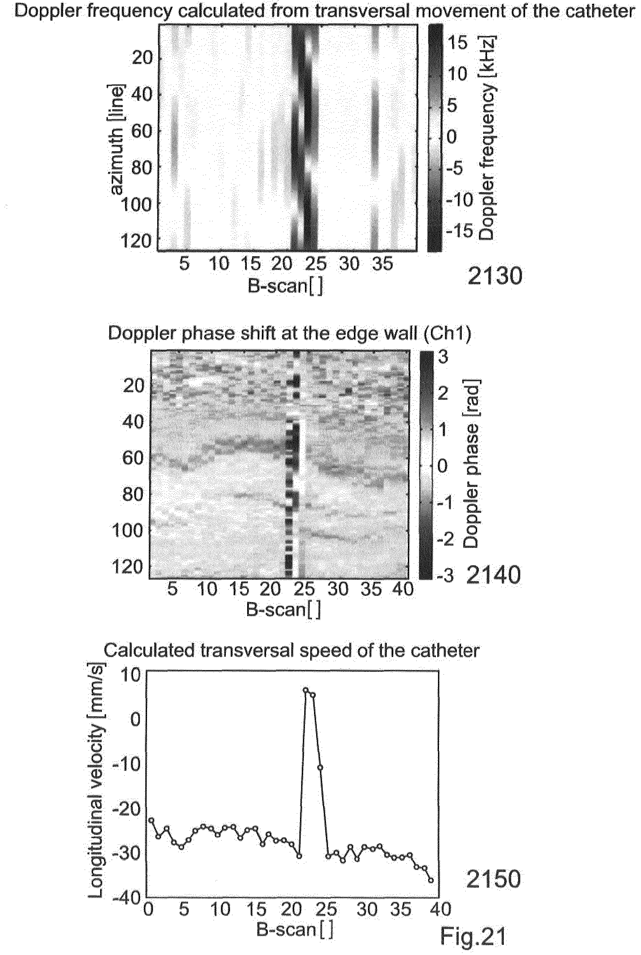

The exemplary systems, methods and computer-accessible medium described herein can facilitate a performance of accurate tracking of the measuring probe. Figure 21 shows a set of illustrations of images and graphs of measurements taken of a probe undergoing stable motion except for a single perturbation during scanning. For example, the exemplary image 2100 is an exemplary frame of reference surface (wall) tracking; the exemplary graph 2110 indicates a transverse position of the probe in time from the reference tracking surface tracking; the exemplary graph 2120 - estimated transverse speed of the probe from the results in the graph 2110; the exemplary image 2130 - estimated Doppler shift from the transverse speed in the graph 2120; the exemplary image 2140 - experimental Doppler shift at the reference surface as a function of frame; the exemplary graph 2150 - residual longitudinal velocity of the probe after subtraction from the graph 2140 of the estimated Doppler shift in the image 2130. The longitudinal velocity of the probe as determined by the exemplary embodiment shows the expected behavior.

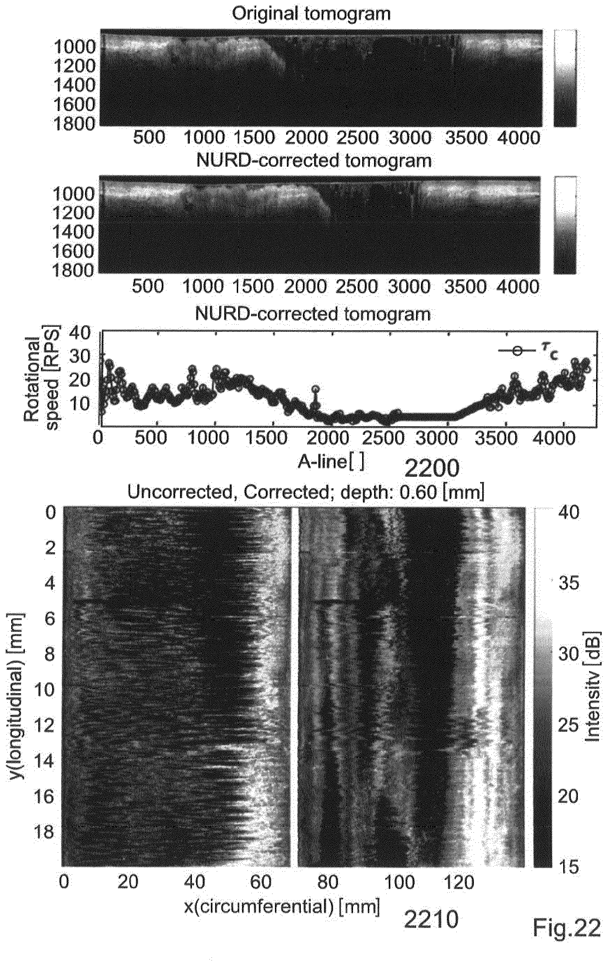

Figure 22 shows a set of illustrations of exemplary rotational probe speed tracking and compensation of image deformation, according to the present disclosure. For example, a set of images/graph 2200 provides an exemplary analysis of the transverse speed of the probe from speckle decorrelation, translation into rotational speed and correction of a deformed image due to NURD. The exemplary images 2210 indicate a projection of a group of 400 images, before and after NURD detection and correction, as explained in the various exemplary embodiments of the methods according to the present disclosure. Although only a few exemplary embodiments of the present disclosure have been described in detail above, those skilled in the art will readily appreciate that many modifications are possible in the exemplary embodiments without materially departing from the novel and non-obvious teachings and advantages of the present disclosure. Accordingly, all such modifications are intended to be included within the scope of the present disclosure as defined in the following claims.

Indeed, the arrangements, systems and methods according to the exemplary embodiments of the present disclosure can be used with and/or implement any OCT system, OFDI system, SD-OCT system or other imaging systems, and for example with those described in International Patent Application PCT/US2004/029148, filed September 8, 2004 which published as International Patent Publication No. WO 2005/047813 on May 26, 2005, U.S. Patent Application No. 11/266,779, filed November 2, 2005 which published as U.S. Patent Publication No. 2006/0093276 on May 4, 2006, and U.S. Patent Application No. 10/501,276, filed July 9, 2004 which published as U.S. Patent Publication No. 2005/0018201 on January 27, 2005, and U.S. Patent Publication No. 2002/0122246, published on May 9, 2002.

It should be understood that the exemplary procedures described herein can be stored on any computer accessible medium, including a hard drive, RAM, ROM, removable disks, CD-ROM, memory sticks, etc., and executed by a processing arrangement and/or computing arrangement which can be and/or include a hardware processors, microprocessor, mini, macro, mainframe, etc., including a plurality and/or combination thereof. In addition, certain terms used in the present disclosure, including the specification, drawings and claims thereof, can be used synonymously in certain instances, including, but not limited to, e.g., data and information. It should be understood that, while these words, and/or other words that can be synonymous to one another, can be used synonymously herein, that there can be instances when such words can be intended to not be used synonymously.

**Claims**

1. An optical frequency domain imaging apparatus comprising:

   a swept source (310) providing a radiation;
   a splitter structure (360a, 370) which separates the radiation into at least one first electro-magnetic radiation directed to a sample (320) and at least one second electro-magnetic radiation directed to a reference arm (340), wherein a frequency of the radiation provided by the swept source is controlled thereby to vary over time; and
   at least one detector second arrangement (350) configured to detect different interferences between at least one third radiation that is a return radiation from the sample which is based on the at least one first radiation and at least one fourth radiation that is a return radiation from the reference arm which is based on the at least one second radiation as a function of time; and
   a computer (380) configured to generate an image, and determine information for measuring a motion within the sample based on a difference between amplitude values of the respective different interferences,
   **characterized in that** the apparatus is configured to:

   determine a Doppler velocity profile in the line of sight,
   determine a total speed profile using speckle decorrelation,
   subtract the Doppler profile from the speckle profile, and
   determine areas with turbulent flow based on the result of the subtraction.

2. The apparatus according to claim 1, wherein the sample is a living body.

3. An optical frequency domain imaging method comprising:

   providing a radiation;
   separating the radiation into at least one first electro-magnetic radiation directed to a sample and at least one second electro-magnetic radiation directed to a reference arm, wherein a frequency of the radiation provided controlled to vary over time;
   detecting different interferences between at least one third radiation that is a return radiation from the sample which is based on the at least one first radiation and at least one fourth radiation that is a return radiation from the reference arm which is based on the at least one second radiation as a function of time;
   generating an image; and
   determining information for measuring a motion within the sample based on a difference between amplitude values of the respective different interferences,
   **characterized in that** the method comprises:

   determining a Doppler velocity profile in the Line of Sight,
   determining a total speed profile using speckle decorrelation,
   subtracting the Doppler profile from the speckle profile, and
   determine areas with turbulent flow based on the result of the subtraction.

4. The apparatus according to claim 1, wherein the determination of the information is based on a speckle decorrelation time by calculating an autocorrelation of the amplitude values as a function of time.

5. The method according to claim 3, wherein the determination of the information is based on a speckle decorrelation time by calculating an autocorrelation of the amplitude values as a function of time.

**Patentansprüche**

1. Optische Frequenzbereichsabbildungsvorrichtung, die Folgendes aufweist:

   eine gewobbelte Quelle (310), die eine Strahlung liefert;
   eine Weichenstruktur (360a, 370), die die Strahlung in mindestens eine erste elektromagnetische Strahlung, die auf eine Probe (320) gerichtet ist, und in mindestens eine zweite elektromagnetische Strahlung, die auf einen Referenzarm (340) gerichtet ist, trennt,
   wobei eine Frequenz der von der gewobbelten Quelle gelieferten Strahlung so gesteuert wird, dass sie über die Zeit variiert; und

   mindestens eine zweite Detektoranordnung (350), die so konfiguriert ist, dass sie unterschiedliche Interferenzen zwischen mindestens einer dritten Strahlung, die eine Rückstrahlung von der Probe ist, die auf der mindestens einen ersten Strahlung basiert, und mindestens einer vierten Strahlung, die eine Rückstrahlung vom Referenzarm ist, die auf der mindestens einen zweiten Strahlung basiert, als Funktion der Zeit erfasst; und
   einen Computer (380), der so konfiguriert ist, dass er ein Bild erzeugt und Informationen zum Messen einer Bewegung innerhalb der Probe auf der Grundlage einer Differenz zwischen Amplitudenwerten der jeweiligen unterschiedlichen Interferenzen bestimmt,

   **dadurch gekennzeichnet, dass** die Vorrichtung konfiguriert ist, um:

   ein Doppler-Geschwindigkeitsprofil in der Sichtlinie zu bestimmen,
   ein Gesamtgeschwindigkeitsprofil unter Verwendung von Speckle-Dekorrelation zu bestimmen,
   das Doppler-Profil von dem Speckle-Profil zu subtrahieren, und
   Bereiche mit turbulenter Strömung basierend auf dem Ergebnis der Subtraktion zu bestimmen.

2. Vorrichtung nach Anspruch 1, wobei die Probe ein lebender Körper ist.

3. Optisches Frequenzbereichsabbildungsverfahren, das Folgendes umfasst:

   Bereitstellen einer Strahlung;
   Trennen der Strahlung in mindestens eine erste elektromagnetische Strahlung, die auf eine Probe gerichtet ist, und mindestens eine zweite elektromagnetische Strahlung, die auf einen Referenzarm gerichtet ist, wobei eine Frequenz der bereitgestellten Strahlung so gesteuert wird, dass sie über die Zeit variiert;
   Erfassen unterschiedlicher Interferenzen zwischen mindestens einer dritten Strahlung, die eine Rückstrahlung von der Probe ist, die auf der mindestens einen ersten Strahlung basiert, und mindestens einer vierten Strahlung, die eine Rückstrahlung vom Referenzarm ist, die auf der mindestens einen zweiten Strahlung basiert, als Funktion der Zeit;
   Erzeugen eines Bildes; und
   Bestimmen von Informationen zur Messung einer Bewegung innerhalb der Probe auf der Grundlage einer Differenz zwischen Amplitudenwerten der jeweiligen unterschiedlichen Interferenzen,

   **dadurch gekennzeichnet, dass** das Verfahren umfasst:

   Bestimmen eines Doppler-Geschwindigkeitsprofils in der Sichtlinie,
   Bestimmen eines Gesamtgeschwindigkeitsprofils unter Verwendung von Speckle-Dekorrelation,
   Subtrahieren des Dopplerprofils von dem Speckleprofil, und
   Bestimmen von Gebieten mit turbulenter Strömung basierend auf dem Ergebnis der Subtraktion.

4. Gerät nach Anspruch 1, wobei die Bestimmung der Information auf einer Speckle-Dekorrelationszeit basiert, indem eine Autokorrelation der Amplitudenwerte als Funktion der Zeit berechnet wird.

5. Verfahren nach Anspruch 3, wobei das Bestimmung der Information auf einer Speckle-Dekorrelationszeit basiert, indem eine Autokorrelation der Amplitudenwerte als Funktion der Zeit berechnet wird.

**Revendications**

1. Appareil d'imagerie de domaine de fréquence optique comprenant :

   une source balayée (310) fournissant un rayonnement ;
   une structure de diviseur (360a, 370) qui sépare le rayonnement en au moins un premier rayonnement électro-magnétique dirigé vers un échantillon (320) et au moins un deuxième rayonnement électro-magnétique dirigé vers un bras de référence (340),
   dans lequel une fréquence du rayonnement fourni par la source balayée est commandée ainsi pour varier au cours du temps ; et
   au moins un ensemble second détecteur (350) configuré pour détecter différentes interférences entre au moins un troisième rayonnement qui est un rayonnement de retour de l'échantillon qui est basé sur l'au moins un premier rayonnement et au moins un quatrième rayonnement qui est un rayonnement de retour du bras de référence qui est basé sur l'au moins un deuxième rayonnement en fonction du temps ; et
   un ordinateur (380) configuré pour produire une image et déterminer les informations pour mesurer un mouvement au sein de l'échantillon sur la base d'une différence entre les valeurs d'amplitude des interférences différentes respectives,
   **caractérisé en ce que** l'appareil est configuré pour :

   déterminer un profil de vitesse Doppler dans la ligne de vue,
   déterminer un profil de vitesse totale en utilisant une décorrélation de bruit,
   soustraire le profil Doppler au profil de bruit, et
   déterminer les zones avec un écoulement turbulent sur la base du résultat de la soustraction.

2. Appareil selon la revendication 1, dans lequel l'échantillon est un corps vivant.

3. Procédé d'imagerie de domaine de fréquence optique comprenant :

   la fourniture d'un rayonnement ;
   la séparation du rayonnement en au moins un premier rayonnement électro-magnétique dirigé vers un échantillon et au moins un deuxième rayonnement électro-magnétique dirigé vers un bras de référence, une fréquence du rayonnement fourni étant commandée pour varier au cours du temps ;
   la détection de différentes interférences entre au moins un troisième rayonnement qui est un rayonnement de retour de l'échantillon qui est basé sur l'au moins un premier rayonnement et au moins un quatrième rayonnement qui est un rayonnement de retour du bras de référence qui est basé sur au moins un deuxième rayonnement en fonction du temps :

      la production d'une image ; et
      la détermination des informations pour mesurer un mouvement au sein de l'échantillon sur la base d'une différence entre les valeurs d'amplitude des interférences différentes respectives,
      **caractérisé en ce que** le procédé comprend :

         la détermination d'un profil de vitesse Doppler dans la ligne de vue,
         la détermination d'un profil de vitesse totale en utilisant une décorrélation de bruit,
         la soustraction du profil Doppler du profil de bruit, et
         la détermination des zones avec un écoulement turbulent sur la base du résultat de la soustraction.

4. Appareil selon la revendication 1, dans lequel la détermination des informations est basée sur un temps de décorrélation de bruit en calculant une autocorrélation des valeurs d'amplitude en fonction du temps.

5. Procédé selon la revendication 3, dans lequel la détermination des informations est basée sur un temps de décorrélation de bruit en calculant une autocorrélation des valeurs d'amplitude en fonction du temps.

Fig. 1 (Prior art)

Fig. 2 (Prior art)

Fig. 3 (Prior art)

Fig. 4 (Prior art)

Fig. 5 (Prior art)

START

Determine/calculate the Fourier transform of each measured A-line — 500

Determine/calculate a phase difference between adjacent A-lines — 510

Determine/calculate the correction for the phase difference based on the reference sample phase — 520

Determine/calculate a corrected absolute phase — 530

Perform a Doppler frequency analysis using the corrected absolute phase — 540

Determine/calculate velocity profile from Doppler frequency analysis — 550

STOP

Fig.6

START

Determine/calculate the
autocorrelation function

600

Determine/calculate the contrast
function for each region

610

Determine/calculate the SNR for
each region

620

Determine/calculate the decorrelation
time for each region

630

Determine/calculate the speckle speed profile
at each point of the sample from all the
above mentioned parameters

640

STOP

Fig.7

800

810

Fig.8

27

Fig.9A

Fig.9B

Fig.9C

Fig.9D

Fig.9E

Fig.9F

Fig.10

Fig.11

Fig.12A

Fig.12B

Fig.12C

Fig.12D

Fig.12E

Fig.12F

Catheter position
inside tube

Fig.13A

Catheter position
inside tube

Fig.13B

Catheter position
inside tube

Fig.13C

Speckle decorrelation
time-averaged flow profile

Fig.13D

Speckle decorrelation
time-averaged flow profile

Fig.13E

Speckle decorrelation
time-averaged flow profile

Fig.13F

Doppler time-averaged flow velocity profile

Fig.13G

Doppler time-averaged flow velocity profile

Fig.13H

Doppler time-averaged flow velocity profile

Fig.13I

Speckle decorrelation speed, Doppler velocity and structural image

Fig.14A          Fig.14D          Fig.14G

Speckle decorrelation speed, Doppler velocity and structural image

Fig.14B          Fig.14E          Fig.14H

Speckle decorrelation speed, Doppler velocity and structural image

Fig.14C          Fig.14F          Fig.14I

START

Perform a minimum of two measurements with different scanning speeds in the direction of interest — 1500

Determine/calculate the inverse decorrelation time profile at each point of the sample — 1510

Repeat for each direction of interest

Determine/calculate the velocity component of interest from the set of measurements — 1520

1530

Determine/calculate the velocity component of interest and/or the speed profiles — 1540

STOP

Fig.15

Fig.16A

Fig.16B

Parabolic GVD

1636

GVD-diversity speckle GVD

1637

GVD-diversity gradient-
corrected flow speed

1638

Fig.16B

1650

1655

Fig.16C

Fig.16C

START

Determine/calculate a Doppler velocity profile and speckle speed profile — 1700

Determine/calculate the axial and longitudinal components of the speed profile based on the Doppler LOS velocity and speckle speed information — 1710

STOP

Fig.17

START

Determine/calculate a Doppler velocity profile — 1800

Determine/calculate a speckle speed profile — 1810

Determine/calculate the differences in the absolute value of the LOS-corrected Doppler profile and the speckle speed profile — 1820

Determine areas with large discrepancies and mark them as turbulent flow — 1830

STOP

Fig.18

START

Determine/calculate a Doppler velocity
profile of a reference surface that
encompasses most/all azimuthal angles
⌐1900

Determine/calculate the transverse velocity
of the probe by analyzing the motion
of the center of gravity of the reference
surface
⌐1910

Subtract the transversal contribution
to the Doppler signal from the previous step
so that the residual Doppler signal corresponds
to pure longitudinal motion
⌐1920

STOP

Fig.19

START

Determine/calculate transverse speed of a refernce surface using speckle decorrelation that encompasses most/all azimuthal angles ⌐2000

Convert transverse speed into rational speed by considering the distance of the surface to the center of rotation ⌐2010

Optionally correct for missing or excess rotation due to inaccuracies in speed estimation ⌐2020

Optionally correct image data with determined angular probe position as a function time ⌐2030

STOP

Fig.20

Wall detection

2100

Center of the catheter as a function of time

2110

Calculated transversal speed of the catheter

2120

Fig.21

Doppler frequency calculated from transversal movement of the catheter

2130

Doppler phase shift at the edge wall (Ch1)

2140

Calculated transversal speed of the catheter

2150

Fig.21

Original tomogram

NURD-corrected tomogram

NURD-corrected tomogram

2200

Uncorrected, Corrected; depth: 0.60 [mm]

2210

Fig.22

# EP 3 111 833 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61933965
- WO 2009137701 A2, COLICE
- WO 20091112 A2
- US 2004029148 W
- WO 2005047813 A
- US 26677905
- US 20060093276 A
- US 50127604
- US 20050018201 A
- US 20020122246 A

**Non-patent literature cited in the description**

- **MURTAZA et al.** *White Paper Signal Processing Overview of Optical Coherence Tomography Systems for Medical Imaging,* 30 June 2016
- **MOTAGHIANNEZAM et al.** *Logarithmic intensity and speckle-based motion contrast methods for human retinal vasculature visualization using swept source optical coherence tomography,* 01 March 2012